# EUROPEAN PATENT SPECIFICATION

(11) **EP 2 920 321 B1**
(45) Date of publication and mention of the grant of the patent: **27.12.2017**
(21) Application number: 13795450.9
(22) Date of filing: 18.11.2013
(51) Int. Cl.: C12Q 1/68

(54) **NUCLEIC ACID ASSAY FOR DIAGNOSING OR MONITORING A PATHOGEN INFECTION IN A BODILY FLUID FROM A SUBJECT TREATED WITH AN ANTI-PATHOGENIC AGENT**
NUKLEINSÄURETEST ZUR DIAGNOSE ODER ÜBERWACHUNG EINER PATHOGENEN INFEKTION IN EINER KÖRPERFLÜSSIGKEIT EINER MIT EINEM ANTI-PATHOGENEN MITTEL BEHANDELTEN PERSON
DOSAGE D'ACIDE NUCLÉIQUE POUR LE DIAGNOSTIC OU LA SURVEILLANCE D'UNE INFECTION PAR UN PATHOGÈNE DANS UN FLUIDE CORPOREL PROVENANT D'UN SUJET TRAITÉ PAR UN AGENT ANTI-PATHOGÈNE

(30) Priority: 16.11.2012 GB 201220662; 16.11.2012 US 201261727210 P
(43) Date of publication of application: 23.09.2015
(73) Proprietor: Alere Technologies GmbH, 07749 Jena (DE)
(72) Inventor: LABUGGER, Ines, 07749 Jena (DE); DEES, Stefan, 07749 Jena (DE); ERMANTRAUT,Eugen, 07749 Jena (DE)
(74) Representative: Huenges, Martin
(86) International application number: PCT/EP2013/074113
(87) International publication number: WO 2014/076286

(56) References cited:
- WO-A1-2013/132443
- WO-A1-2013/192302
- WO-A2-2006/088895
- WO-A2-2011/106536
- MURDOCH D R ET AL: "Use of the polymerase chain reaction to detect Legionella DNA in urine and serum samples from patients with pneumonia", CLINICAL INFECTIOUS DISEASES, THE UNIVERSITY OF CHICAGO PRESS, CHICAGO, IL, US, vol. 23, no. 3, 1 September 1996 (1996-09-01), pages 475-480, XP008029295, ISSN: 1058-4838
- TAKASHI TAKAHASHI ET AL: "Prolonged Mycoplasma pneumoniae infection in an elderly patient with community-acquired pneumonia", JOURNAL OF INFECTION AND CHEMOTHERAPY, vol. 15, no. 4, 1 August 2009 (2009-08-01) , pages 243-247, XP055094107, ISSN: 1341-321X, DOI: 10.1007/s10156-009-0692-x
- VAN DER HEIJDEN INEKE M. ET AL: "Detection of bacterial DNA in serial synovial samples obtained during antibiotic treatment from patients with septic arthritis", ARTHRITIS & RHEUMATISM, vol. 42, no. 10, 1 October 1999 (1999-10-01), pages 2198-2203, XP055094221, ISSN: 0004-3591, DOI: 10.1002/1529-0131(199910)42:10<2198::AID-A NR23>3.0.CO;2-N
- MARTINEAU F ET AL: "Development of a rapid PCR assay specific for Staphylococcus saprophyticus and application to direct detection from urine samples", JOURNAL OF CLINICAL MICROBIOLOGY, AMERICAN SOCIETY FOR MICROBIOLOGY, US, vol. 38, no. 9, 1 September 2000 (2000-09-01), pages 3280-3284, XP002239983, ISSN: 0095-1137
- HEININGER A ET AL: "PCR and Blood Culture for Detection of Escherichia coli Bacteremia in Rats", Journal of Clinical Microbiology, vol. 37, no. 8 1 August 1999 (1999-08-01), pages 2479-2482, XP055094238, Retrieved from the Internet: URL:http://ukpmc.ac.uk/articlerender.cgi?a rtid=245547 [retrieved on 2013-12-18]
- ESPY: "Real-time PCR in clinical microbiology applications for routine laboratory testing.", CLINICAL MICROBIOLOGY REVIEWS, vol. 19, no. 1, 1 January 2006 (2006-01-01), pages 165-256, XP055038471, ISSN: 0893-8512
- ABBADI S ET AL: "Strain differentiation of Mycobacterium tuberculosis complex isolated from sputum of pulmonary tuberculosis patients", INTERNATIONAL JOURNAL OF INFECTIOUS DISEASES, INTERNATIONAL SOCIETY FOR INFECTIOUS DISEASES, HAMILTON, CA, vol. 13, no. 2, 1 March 2009 (2009-03-01), pages 236-242, XP025995470, ISSN: 1201-9712, DOI: 10.1016/J.IJID.2008.06.020 [retrieved on 2008-10-05]

## Description

### Technical Field

The invention relates to the field of detecting and/or diagnosing the presence and/or the genotype of a pathogen in a subject based on the presence of a polynucleotide in a bodily fluid of said subject. In the bodily fluid, the amount of the polynucleotide which may be of pathogen origin is increased due to the effect of an anti-pathogenic agent which has been administered to the subject before.

### Background

In many diseases, the patient very often shows symptoms which may not be clearly assigned to a specific disease. As a consequence, an effective therapy may not be started immediately but first different therapies are applied in a try-and-error manner to get more information about the disease and the reasons for it. In many cases, a patient's condition may deteriorate significantly or even become life-threatening. On the other hand, the administration of an antibiotic or other drug which is not useful can cause severe side effects and thereby additionally weaken the patient. In best cases, the wrong or unspecific medication is only unnecessary and has neither positive nor negative effect. Nevertheless, such medication is still a cost factor in the health system. Another problem which arises from unspecific administration of antibiotics is the generation of multi-resistant bacterial strains. These multi-resistant strains are a severe health risk in particular in clinics and are extremely dangerous to people having a weak immune system, such as children, older people or people who have a genetic or acquired immune deficiency.

Standard therapies are often simply based on an assumption of the disease from the symptoms of the patient and subsequent standard administration of an antibiotic. After this choice of an antibiotic, mostly only the symptoms of the patient are monitored in order to estimate whether they disappear, stay the same or intensify. Depending on this monitoring, the medication is continued, the dose regimen is adapted or another antibiotic is tested. However, this try-and-error procedure is time consuming and often less optimal.

Even if the disease can be identified with virtual certainty already from the symptoms of a patient, a diagnosis of the specific pathogen causing the disease may be useful for treatment design and medication.

If a diagnostic test is performed in order to determine the specific pathogen being responsible for a patient's clinical picture and/or to monitor the course of the disease and the infection, this may be done using different tests ranging from classical microbiology tests, such as culturing the pathogen/microorganism and subsequent pathogen or microbiologic analysis, over immunological assays being based on the detection of antigens, antibodies or infectious agents to assays on molecular basis which detect the presence of pathogen polynucleotides in a sample.

On a molecular basis, polynucleotides may be detected in bodily fluids which originate from the pathogen itself. Without intention to be bound by a particular theory, the appearance of polynucleotides from pathogens is believed to be a result of fragmentation and degradation of DNA due to cell death. In a first step, this DNA or these polynucleotides enter into blood circulation (hereinafter also referred to as plasma polynucleotides). Hence, polynucleotides present in blood circulation of a subject may be detected in the blood, serum or plasma of said subject for a certain period of time. Part of these circulating polynucleotides is degraded completely in the circulatory system, subsequently. However, another part of the plasma or circulatory polynucleotides crosses the kidney barrier and ends up in the urine of the subject. Concentrated in urine, the now so-called renal or transrenal polynucleotides may be detected in urine. Hence, plasma or renal or transrenal polynucleotides may be used as diagnostic markers to detect the presence of non-subject but pathogen polynucleotides in a bodily fluid of a subject.

The appearance of DNA from necrotic or apoptotic cells of different tissues throughout the body in plasma is described in the art (Lichtenstein AV et al., 2006, Annals of the New York Academy of Sciences, 945:239-249). This phenomenon has also been published for tumor DNA by Sozzi G et al. (2001, Cancer Research, 61:4675-4678) who identified differences in plasma DNA level and molecular characterization in lung cancer patients compared to healthy individuals.

As already mentioned above, a small portion of plasma circulating polynucleotides may leave the blood plasma via the kidney barrier and end up in the urine of a patient. Su *et al.* have examined urine probes of healthy volunteers and patients having colorectal tumors or pancreatic cancer in order to evaluate the use of urine in the diagnostics of cancer (Su Y-H et al., 2004, Annals of the New York Academy of Sciences, 1022:81-89). EP 2 351 857 A1 and US 2010/0068711 A1 describe compositions and methods for detecting pathogen specific nucleic acids in urine without specific detection-related prior treatment of the patient.

Beside the diagnosis of cancer and fetal diseases also the determination of the male gender of a fetus is possible by the analysis of circulating polynucleotides (Lichtenstein AV et al., 2006, Annals of the New York Academy of Sciences, 945:239-249). In recent years, the presence of polynucleotides in plasma or urine as a tool for the detection of infectious diseases has come into the focus of attention.

Fu *et al.* for example describe the analysis of blood microRNAs (miRNAs) in patients with active pulmonary tuberculosis (Fu Y et al., 2011, Journal of Clinical Microbiology, 49:4246-4251). Using microarray-based expression profiling followed by real-time quantitative PCR validation variations in miRNA regulation in healthy volunteers and infection patients have been investigated. Thereby, a number of pathways have been identified to be involved in acute-phase response, inflammatory response and the regulation of the cytoskeleton and thus revealed early pathogen-host interactions. Green et al. have discussed nucleic acid amplification techniques for identification of *Mycobacterium tuberculosis* (*M. tuberculosis*) in respiratory samples, such as sputum, bronchoalveolar lavage or oral washes (Green C. et al., 2009, The Lancet Infectious Diseases, 9(8):505-511).

However, the detection and thus diagnosing of infectious diseases is often considered insecure and not reliable, as expressed in a review of T.Tuuminen (2012, Frontiers in Immunology, 3:1-6) who refers to an article of Cook et al. (2005, Ann. Intern. Med. 142:914-925), wherein PCR analysis has not been recommended for detection of gonococcal infection in urine. The problems associated with the isolation of comparatively short polynucleotides being present in a too low concentration in solution is further discussed by Lichtenstein AV et al. (2006, Annals of the New York Academy of Sciences, 945:239-249). In summary, plasma and renal polynucleotides indicative of pathogen infection may be present in very low concentration, since only a small portion of these analytes escapes degradation in the blood. An even smaller portion can further cross the kidney barrier and appear in urine. In general, the concentration of polynucleotides in plasma may be about 10 - 100 ng per mL (Lichtenstein AV et al., 2006, Annals of the New York Academy of Sciences, 945:239-249; Xue X et al., 2009, Clinica Chimica Acta, 404:100-104). Such a low concentration of these relatively short polynucleotides is often below the sensitivity of the isolation method and amplification and detection may not be successful.
WO 2006/088895 A2 discloses methods to identify drug-resistant non-viral pathogenic infections by detecting nucleic acids in urine after the subject has been treated for the infection by a drug. The disclosed method is based on detection in urine specimens from patients of cell-free M. tuberculosis DNA fragments originating from sites of infection outside the urogenital tract.
WO 2011/106536 A2 discloses a method of distinguishing between drug-sensitive and drug-resistant pathogens such as *M. tuberculosis*, comprising detecting early transcriptional responses to drug exposure to determine changes in the mRNA expression profiles of the pathogen. This assessment is done in vitro and using a polynucleotide released by a living pathogenic cell.

### Summary

It is thus an objective of the present disclosure to provide a method for diagnosing a disease which allows a reliable and quick determination of the microorganism or pathogen which is responsible for the symptoms and disease of the patient via detection of a polynucleotide in a bodily fluid of the patient such as the blood or plasma or the urine of the patient.

Another objective of the present disclosure is the estimation of the required anti-pathogenic agent or antibiotic and/or the required dosage regimen regarding amount of antibiotic and length of treatment from an early response peak of polynucleotides in plasma and/or urine which may be detected after an initial treatment with antibiotic.

It has been found that the concentration of polynucleotides originating from a pathogen, optionally from bacteria or other microorganisms susceptible to anti-pathogenic agents, such as antibiotics, may increase significantly after administration of an anti-pathogenic agent or antibiotic. Hence, the administration of an anti-pathogenic agent or antibiotic may increase the amount of polynucleotide in a bodily fluid, such as in plasma and/or in urine, so that isolation and subsequent detection is possible. For example, the administration of an anti-pathogenic agent or antibiotic may increase the amount of polynucleotides in plasma and urine, subsequently, so that detection is possible at all or in a much better reliability. In particular, the administration of an anti-pathogenic agent or antibiotic may increase the amount of pathogen polynucleotides in a bodily fluid such as blood or urine in which the pathogen is typically not present.

This may be of advantage if the symptoms of a patient are not so clear that the disease or the causing bacterium or pathogen could be identified in a manner sufficient enough to start a suitable therapy.

Another advantage of the method disclosed herein may be that the initial administration of an anti-pathogenic agent or antibiotic which is likely to cure the disease being assumed from patient's symptoms, immediately has a treating or curing effect on the patient. In case of a very unspecific clinical picture, the administration of a broadband antibiotic may further help to identify the pathogen due to sequence analysis of the polynucleotide. Thereby, even if a disease is determined with virtual certainty, the method disclosed herein may help to identify the specific pathogen or bacteria strain or may detect mutations in the DNA sequence to identify mutated strains thereof.

Also disclosed is a method for identifying the most optimal anti-pathogenic agent or antibiotic and/or for assessing the suitability of an anti-pathogenic agent or antibiotic for a specific infection treatment and dosage regimen of said anti-pathogenic agent or antibiotic depending on an early response peak. This early response peak denotes a significant increase in the polynucleotide concentration detectable in a bodily fluid, such as plasma and/or urine, after the administration of the anti-pathogenic agent or antibiotic. The peak may appear, *e.g.* 1 to 10 days after treatment initiation or administration of the anti-pathogenic agent or antibiotic. From the height of the peak, the peak area and/or the form of the curve, it is possible to determine certain parameters related to the infection or presence of a pathogen, such as, but not limited to, the effectiveness of the anti-pathogenic agent with respect to the pathogen causing the symptoms and/or disease of the patient, and also with respect of the intensity of the infection. Thereby, treatment conditions such as dosage regimen and/or length of treatment may be estimated.

For instance, the higher the peak, the more effective may be the anti-pathogenic agent or antibiotic towards the pathogen or bacterium, respectively. The height of the peak may also reflect the amount of pathogen present in the body and thus the intensity of the pathogen infection. If the peak is relatively low, this may indicate a low effectiveness of treatment with the anti-pathogenic agent or antibiotic or a low amount of pathogen/bacteria in the body. In some embodiments, the peak area increases with the peak height, however, the peak may be very discrete and steep or comparatively flat and broad. From the form of the peak it may be differentiated, for instance, between effectiveness of treatment with the anti-pathogenic agent or antibiotic and low or higher pathogen concentration in the body. The form of the peak may be characterized by the peak height in relation to peak width. A wider peak in relation to the peak height may indicate a lower effectiveness of treatment with the anti-pathogenic agent as polynucleotides are released to the plasma and urine over a longer period of time. In this case, the anti-pathogenic agent may be changed or the dosage of the used anti-pathogenic agent may be increased. A relatively steep and pronounced peak may indicate a high effectiveness of treatment with the used anti-pathogenic agent or antibiotic. In this case, the anti-pathogenic agent may not be changed. Optionally, the concentration or dosage of the used anti-pathogenic agent may be altered, e.g. decreased depending on the height of the peak. It may be regarded to give satisfactory or even best therapeutic results if the plasma and urine concentration of polynucleotide is monitored until no polynucleotide may be detected anymore or at least until no symptoms are shown by the patient anymore. In the end, the physician in charge can decide about a patient's treatment and anti-pathogenic agent/antibiotic and additional treatment may be chosen according to the physician's evaluation of a patient's health status.

In some embodiments, the method combines more than one positive aspect: accurate, secure and/or safe detection and determination of the pathogen/bacterium which causes the disease and immediate therapy of the patient. Additionally, the treatment, choice of anti-pathogenic agent/antibiotic and dosage regimen of a patient may be determined individually according to the specific clinical picture of the patient. For example, a method is provided for determining the kind of disease, in particular diagnosing the type of infection, the effectiveness of the anti-pathogenic agent or antibiotic and/or the method may also provide for genotyping of the pathogen, such as microorganism or bacteria.

The above mentioned and further objectives can be solved by the disclosure manifested in the claims and embodiments described herein.

In one embodiment, a method for detecting the presence of a polynucleotide in a bodily fluid obtained from a subject treated with an anti-pathogenic agent is disclosed comprising
- isolating the polynucleotide from the bodily fluid,
- amplifying the polynucleotide, and
- determining the polynucleotide,
wherein the polynucleotide is a pathogen polynucleotide.

In another embodiment, a method for diagnosing a pathogen infection in a subject is disclosed comprising
- administering an anti-pathogenic agent to the subject,
- obtaining a bodily fluid from the subject,
- isolating a polynucleotide indicative for the pathogen infection from the bodily fluid,
- amplifying the polynucleotide,
- detecting the polynucleotide, and/or
- determining the pathogen infection.

In another embodiment, a method for detecting a pathogen infection in a subject treated with an anti-pathogenic agent is disclosed comprising
- isolating a polynucleotide indicative for the pathogen infection from a bodily fluid obtained from the subject,
- amplifying the polynucleotide, and
- detecting the polynucleotide.

In another embodiment, a method for detecting the presence and/or genotype of a pathogen in a bodily fluid is disclosed comprising
- isolating a polynucleotide indicative of the presence and/or genotype of the pathogen from the bodily fluid, wherein the bodily fluid is obtained from a subject after administration of an anti-pathogenic agent,
- amplifying the polynucleotide,
- determining the polynucleotide indicative of the presence and/or genotype of the pathogen.

In another embodiment, a method of diagnosing or monitoring a pathogen infection in a subject comprises:
determining a value indicative of the presence and/or amount of a polynucleotide indicative of the pathogen infection in a sample of a bodily fluid obtained from the subject after administration of an anti-pathogenic agent,
comparing the value to a baseline value indicative of the presence and/or amount of the polynucleotide in a sample of the bodily fluid before administration of an anti-pathogenic agent, wherein a value higher than the baseline value is indicative of a pathogen infection.

The bodily fluid may be selected from bodily fluids which do not contain the pathogen.

The methods may further comprise a step of isolating the polynucleotide indicative of the pathogen infection from a sample of the bodily fluid obtained from the subject after administration of the anti-pathogenic agent.

Typically, the baseline value can be determined based on a sample of the body fluid obtained from the same subject or from a population of subjects.

In some embodiments, the method comprises:
- isolating a polynucleotide indicative of the pathogen infection from a first sample of a bodily fluid obtained from the subject before administration of an anti-pathogenic agent to the subject,
- determining a baseline value indicative of the presence and/or amount of the polynucleotide in the first sample of the bodily fluid,
- isolating the polynucleotide indicative for the pathogen infection from a second sample of the bodily fluid obtained from the subject after administration of the anti-pathogenic agent,
- determining a value indicative of the presence and/or amount of the polynucleotide in the second sample of the bodily fluid, and
- comparing the value to the baseline value, wherein a value higher than the baseline value is indicative of a pathogen infection.

The methods described above may further comprise a step of determining the genotype of the pathogen.

In one embodiment, a method for detecting a pathogen infection in a subject is disclosed comprising
- isolating a polynucleotide indicative of the pathogen infection from a first sample of a bodily fluid obtained from the subject prior to administering an anti-pathogenic agent to the subject,
- determining a first value indicative of the presence and/or amount of the polynucleotide in the first sample of the bodily fluid,
- isolating the polynucleotide indicative for the pathogen infection from a second sample of the bodily fluid obtained from the subject after administration of the anti-pathogenic agent,
- determining a second value indicative of the presence and/or amount of the polynucleotide in the second sample of the bodily fluid,
- comparing the second value with the first value, and/or
- determining the pathogen infection if the second value is higher than the first value.

In one embodiment, a method for detecting the presence and/or genotype of a pathogen in a bodily fluid is disclosed comprising
- isolating a polynucleotide indicative of the presence and/or genotype of the pathogen from a first sample of the bodily fluid obtained from a subject prior to administering an anti-pathogenic agent to the subject,
- determining a first value indicative of the presence and/or amount of the polynucleotide in the first sample of the bodily fluid,
- isolating the polynucleotide indicative of the presence and/or genotype of the pathogen from a second sample of the bodily fluid obtained from the subject after administration of the anti-pathogenic agent,
- determining a second value indicative of the presence and/or amount of the polynucleotide in the second sample of the bodily fluid,
- comparing the second value with the first value,
- determining the sequence of the polynucleotide, and/or
- determining the presence and/or genotype of the pathogen before and after the anti-pathogenic agent was administered to the subject.

In one embodiment, a method for diagnosing a pathogen infection in a subject is disclosed comprising
- obtaining a first sample of a bodily fluid from the subject,
- isolating a polynucleotide indicative of the pathogen infection from the first sample of the bodily fluid,
- determining a first value indicative of the concentration and/or amount of the polynucleotide in the first sample of the bodily fluid,
- administering an anti-pathogenic agent to the subject,
- obtaining a second sample of the bodily fluid from the subject,
- isolating the polynucleotide indicative of the pathogen infection from the second sample of the bodily fluid,
- determining a second value indicative of the concentration and/or amount of the polynucleotide in the second sample of the bodily fluid,
- comparing the second value with the first value, and/or
- determining the pathogen infection if the second value is higher than the first value.

In one embodiment, a method of monitoring the presence and/or amount of a polynucleotide in a bodily fluid is disclosed comprising
- isolating the polynucleotide from a first sample of the bodily fluid obtained from a subject prior to administering an anti-pathogenic agent to the subject,
- determining a first value indicative of the presence and/or amount of the polynucleotide in the first sample of the bodily fluid,
- isolating the polynucleotide from a second sample of the bodily fluid obtained from the subject after administration of the anti-pathogenic agent,
- determining a second value indicative of the presence and/or amount of the polynucleotide in the second sample of the bodily fluid, and/or
- comparing the second value with the first value,
wherein the polynucleotide is a pathogen polynucleotide.

In all embodiments described above, the order of the steps comprised in the method may be in any order or in the order given.

Also disclosed is a method of any of the embodiments described above, wherein the subject was treated with the anti-pathogenic agent 1 to 10 days prior to obtaining and/or isolating the polynucleotide from the bodily fluid.

Also disclosed is a method of any of the embodiments described above, wherein the subject is treated with the anti-pathogenic agent 2 to 8 days, optionally 3 to 6 days, prior to obtaining and/or isolating the polynucleotide from the bodily fluid.

Also disclosed is a method of any of the embodiments described above, wherein the polynucleotide is a pathogen polynucleotide, optionally a microbial polynucleotide.

Also disclosed is a method of any of the embodiments described above, wherein the pathogen polynucleotide is a bacterial polynucleotide.

Also disclosed is a method of any of the embodiments described above, wherein the polynucleotide is DNA or RNA.

Also disclosed is a method of any of the embodiments described above, wherein the DNA or RNA is renal or transrenal DNA, renal or transrenal RNA, or microRNA.

Also disclosed is a method of any of the embodiments described above, wherein the polynucleotide is derivable from a pathogen selected from the group consisting of *Mycobacterium tuberculosis, Mycobacterium bovis, Mycobacterium leprae, Mycobacterium africanum*, *Helicobacter pylori, Bacillus anthracis, Chlamydia trachomatis, Neisseria gonorrhoeae*, *Neisseria meningitidis, Streptococcus pneumonia, Chlamydia pneumonia, Pseudomonas aeruginosa, Escherichia coli, Klebsiella species*, *Enterobacter species*, *Proteus species* and other *Enterobacteria, Staphylococcus aureus, Cryptococcus neoformans, Histoplasma capsulatum, Aspergillus spp, Schistosoma mansoni, Schistosoma haemotobium*, *Legionella pneumophila*, *Plasmodium species*, and combinations thereof.

Also disclosed is a method of any of the embodiments described above, wherein the bodily fluid is selected from the group consisting of urine, bronchoalveolar lavage, lacrimal fluid, lymphatic fluid, blood, plasma, sputum or serum. Typically, the bodily fluid is selected from the group consisting of urine, bronchoalveolar lavage, blood (such as plasma or serum), and sputum.

Also disclosed is a method of any of the embodiments described above, wherein the subject is a human or animal.

Also disclosed is a method of any of the embodiments described above, wherein the subject was treated with the anti-pathogenic agent 1 to 10 days, optionally 2 to 8 days, before the second sample of the bodily fluid was obtained from the subject.

Also disclosed is a method of any of the embodiments described above, wherein the subject was treated with the anti-pathogenic agent 1 to 10 days, optionally 2 to 8 days, prior to obtaining the second sample of the bodily fluid and/or isolating the polynucleotide from the second sample of the bodily fluid.

Also disclosed is a method of any of the embodiments described above, wherein the anti-pathogenic agent is an antibiotic, optionally the antibiotic is a natural antibiotic or an antibiotic analog, optionally the antibiotic analog is a synthetic antibiotic or a semi-synthetic antibiotic.

Also disclosed is a method of any of the embodiments described above, wherein the anti-pathogenic agent is effective against a pathogen of the genus *Mycobacterium, Helicobacter, Bacillus, Leishmania, Chlamydia, Neisseria, Streptococcus, Pseudomonas, Escherichia*, *Klebsiella, Enterobacter, Proteus, Enterobacteria*, *Staphylococcus, Cryptococcus, Histoplasma, Aspergillus, Schistosoma, Legionella, Plasmodium*, and combinations thereof.

Also disclosed is a method of any of the embodiments described above, wherein the anti-pathogenic agent is selected from the group consisting of isoniazid, rifampicin, pyrazinamide, ethambutol, streptomycin, dapsone, clofazimine, clarithromycin, amoxicillin, doxycycline, erythromycin, vancomycin, silver nitrate, penicillins, tetracyclines, cefotaxime, ceftriaxone, aminopenicillins, cephalosporines, macrolides, azithromycin, acylaminopenicillins, aminoglycosides, carbapenems, fluoroquinolones, flucloxacillin, teicoplanin, amphotericin B, fluconazol, azoles, posacanazol, echinocandines, praziquantel, oxamniquin, quinine, mefloquine, resoquine, gentamicin, clindamycin, and combinations thereof.

Also disclosed is a method of any of the embodiments described above, wherein the pathogen is susceptible to the anti-pathogenic agent, e.g. is not resistant against the anti-pathogenic agent.

Also disclosed is a method of any of the embodiments described above, wherein the pathogen belongs to a genus selected from the group consisting of *Mycobacterium, Helicobacter, Bacillus, Leishmania, Chlamydia, Neisseria, Streptococcus, Pseudomonas, Escherichia, Klebsiella, Enterobacter, Proteus, Enterobacteria, Staphylococcus, Cryptococcus, Histoplasma, Aspergillus, Schistosoma, Legionella, Plasmodium,* and combinations thereof.

Also disclosed is a method of any of the embodiments described above, wherein the pathogen is a microorganism, optionally a bacterium.

Also disclosed is a method of any of the embodiments described above, wherein the pathogen infection is selected from the group consisting of tuberculosis, pulmonary tuberculosis, paediatric, extrapulmonary or HIV-associated tuberculosis, leprosy, chronic gastritis, gastric ulcers, peptic ulcers, anthrax, gonorrhoea, orchitis, conjunctivitis, pharyngitis, proctitis, urethritis, prostatitis, bacterial meningitis, infertility, meningococcal septicaemia, community acquired pneumonia, sepsis, meningitis, urinary tract infections, pyelonephritis, pneumonia, nosocomial or ventilator-associated pneumonia, lung infections, histoplasmosis, disseminated mycoses, schistosomiasis, malaria, and combinations thereof.

Further disclosed is an assay or kit for performing the method of to any one of the preceding claims comprising
- a device or means for isolating the polynucleotide from the bodily fluid,
- a device or means for amplifying the polynucleotide, and
- a device or means for detecting the polynucleotide.

Additionally, disclosed is the use of an anti-pathogenic agent for increasing a value indicative of the concentration and/or amount of a pathogen polynucleotide in a bodily fluid of a subject.

### Brief Description of the Drawings

- **Figure 1:**: Drug-induced kinetic of transrenal (tr) DNA secretion in unfractionated urine from lung TB patient 22. Excretion of mycobacterial trDNA upon initiation of antibiotic treatment.
- **Figure 2:**: Detectable amount of circulating DNA in urine and plasma of TB patient 22 after treatment initiation (same patient as in Figure 1 with 1 mL urine and 1 mL plasma).
- **Figure 3:**: Drug-induced kinetic of transrenal (tr) DNA secretion in unfractionated urine from lung TB patient 50. Excretion of mycobacterial trDNA upon initiation of antibiotic treatment.
- **Figure 4:**: Detectable amount of circulating DNA in urine and plasma (Figure 4A) and plasma (Figure 4B, same data as in Figure 4A but different scale) of TB patient 50 after treatment initiation (same patient as in Figure 3 with 1 mL urine and 1 mL plasma).
- **Figure 5:**: Drug-induced kinetic of transrenal (tr) DNA secretion in unfractionated urine from lung TB patient 56. Excretion of mycobacterial trDNA upon initiation of antibiotic treatment.
- **Figure 6:**: Detectable amount of circulating DNA in urine and plasma of TB patient 56 after treatment initiation (same patient as in Figure 5 with 1 mL urine and 1 mL plasma).
- **Figure 7:**: Detectable amount of circulating DNA in urine of TB patient 21 after treatment initiation and treatment modification at day 63 (1 mL urine).
- **Figure 8:**: Detectable amount of circulating DNA in urine of TB patient 23 after treatment initiation and treatment modification at day 38 (1 mL urine).

### Detailed Description

Where the term "comprise" or "comprising" is used in the present description and claims, it does not exclude other elements or steps. For the purpose of the present disclosure, the term "consisting of' is considered to be an optional embodiment of the term "comprising of'. If hereinafter a group is defined to comprise at least a certain number of embodiments, this is also to be understood to disclose a group which optionally consists only of these embodiments.

Where an indefinite or a definite article is used when referring to a singular noun, e.g. "a" or "an", "the", this includes a plural form of that noun unless specifically stated. For example, when a polynucleotide is mentioned, this is also to be understood as polynucleotides. *Vice versa*, when the plural form of a noun is used it refers also to the singular form.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of the terms will be given below in the context of which the terms are used.

Analysis of biological samples may include determining whether one or more polynucleotides (for instance, a DNA, RNA, mRNA, or rRNA) are present in the sample. For example, one may analyze a sample to determine whether a polynucleotide indicative of the presence of a particular pathogen is present.

A "disease" in the sense of the disclosure is any condition of a subject, also referred to as patient, human or animal, that is altered in comparison to the condition of a subject which is regarded healthy.

The term "infection" is to be understood according to its general meaning in the field of medicine and denotes a state in which one or more pathogens, microorganisms or bacteria are present in an organism and may have already or may subsequently lead to a clinical picture which may be regarded as disease or unhealthy. However, a subject may also be infected with a pathogen, such as microorganism or bacterium, but does not show any change in the clinical picture or show symptoms of a disease. Such an infection is denoted as occult or asymptomatic infection. The method disclosed herein may be particularly useful in detecting infections in a subject. Hence, in case of a pathogen, bacterial or microbial infection a pathogen, bacterium or microorganism, respectively, is present in a subject. In this context, it is well understood by a person skilled in the art that detecting the "presence" of polynucleotide in a bodily fluid of a subject may be used to detect a bacterial or microbial infection of the subject from which the bodily fluid was obtained.

The terms "pathogen infection", "microbial infection" and "bacterial infection" are to be understood according to their general meaning in the field of medicine and microbiology and is in general regarded as a disease which is caused by a pathogen, microorganism or bacterium, respectively. Optionally, the pathogen infection is a microbial infection, optionally a bacterial infection.

"Diagnosing" an infection denotes the determination of a specific disease of a subject by analyzing the symptoms, clinical condition and optionally also other factors, such as laboratory and microscopic indications. The method disclosed herein may be explicitly useful for diagnosing a pathogen infection, such as a microbial or bacterial infection, by isolating a polynucleotide being indicative of an infection of the subject from a bodily fluid of said subject being optionally treated with an anti-pathogenic agent or antibiotic before, amplifying the polynucleotide, detecting the polynucleotide and thereby, determining and diagnosing the pathogen infection of the subject.

"Tuberculosis" (TB) is generally known as an infectious disease of the lung caused by bacteria of the genus *Mycobacterium, e.g. Mycobacterium tuberculosis.* Forms of tuberculosis comprise for example pulmonary tuberculosis, paediatric tuberculosis, extrapulmonary tuberculosis or HIV-associated tuberculosis.

The terms "pathogen" and "microorganism" are to be understood according to its general meaning in the field of medicine, microbiology. Hence, the term microorganism is generally used to denote bacteria, yeast, fungi, single-celled plants or other eukaryotes and prokaryotes. A "pathogen" generally denotes an organism, such as a microorganism or bacteria, which may have an adverse effect on a subject, e.g. human or animal, and may cause a disease or a clinical picture which may be regarded as not healthy. A preferred pathogen is a microorganism, optionally a bacterium.

The terms "bacteria" or "bacterium" denote a microorganism and are to be understood according to their general meaning in the field of microbiology and biology. Both terms further include all microorganisms which are susceptible to a treatment with an antibiotic or antibiotic analog. A bacterium is a typical pathogen. "Susceptible to a treatment with an anti-pathogenic agent or antibiotic" denotes that the pathogen or microorganism either does not grow/divide any longer after contact with the anti-pathogenic agent or antibiotic or dies after contact with the anti-pathogenic agent or antibiotic.

The term "anti-pathogenic agent" may refer to a single anti-pathogenic agent or to a combination of anti-pathogenic agents such as a combination of a plurality of anti-pathogenic agents.

The terms "genus" of a pathogen or microorganism and "genotype" are to be understood according to their general meaning in the field of biology, micro- and molecular biology. In general, genus is a rank in biological classification and the genotype of is the specific genetic constitution of an organism. By determining the genotype or by "genotyping", the exact species of an organism, in particular of a pathogen, such as a microorganism or bacterium, may be determined.

The term "polynucleotide" is to be understood according to its general meaning in the field of molecular biology and genetics. The term "polynucleotide" particularly comprises nucleic acid sequences having a minimum of 10 bp and comprising or may consist of variable compositions of the standard bases adenine, guanine, cytosine, and thymine in case of DNA or uracil in case of RNA. The terms "polynucleotide", "nucleic acid" or "nucleic acid molecule" are interchangeable.

A "pathogen polynucleotide" or a "polynucleotide of pathogen origin" is a polynucleotide which is derivable or derived or originates from a pathogen. An exemplary pathogen polynucleotide is a "microbial polynucleotide" which is a polynucleotide which is derivable or derived or originates from a microorganism. An exemplary microbial polynucleotide is a "bacterial polynucleotide" which is derivable or derived or originates from a bacterium. Hence, a polynucleotide which is derivable or derived or originates from a pathogen, microorganism or from a bacterium originates from a pathogen cell or microbial or bacterial cell and may for example be released from the cell after the death of the pathogen, microorganism or bacterium and may then circulate in the blood plasma and/or cross the kidney barrier to end up in urine as renal or transrenal polynucleotide.

"DNA" denotes deoxyribonucleic acid and "RNA" denotes a ribonucleic acid. Both terms are to be understood according to their general meaning in the field of molecular biology and genetics. "PNA" denotes a peptide nucleic acid and denotes an artificially synthesized polymer which resembles DNA and RNA. Further information on PNA may be taken from P.E. Nielsen (2008, Sci Am., 299(6):64-71).

"Circulating nucleic acids/ circulating polynucleotides" or "plasma polynucleotides" or "cell-free polynucleotides" comprise nucleic acid molecules or polynucleotides (*e.g.* DNA or RNA) which circulate or are present a bodily fluid, such as in the lymphatic system, in the blood stream or blood circulation of a subject, preferably of a human or animal. Circulating DNA/RNA may also be denoted as "plasma DNA" or "plasma RNA" ("plasma polynucleotides") or "lymphatic polynucleotides". Circulating polynucleotides may be detected in the method disclosed herein.

A "renal" (r) or "transrenal" (tr) polynucleotide denotes a polynucleotide which has passed the kidney from blood plasma and may then be present in urine. A renal or transrenal polynucleotide may be DNA (rDNA or trDNA) or RNA (rRNA or trRNA) or any other form of a polynucleotide. It is believed that a polynucleotide which is released from a dead cell, such as a pathogen or bacterial cell, may not be degraded, or at least not completely, so that an amount of the polynucleotide circulates e.g. in the blood plasma. From there it may cross the kidney and appear in urine. In urine, it finally leaves the body in a non-invasive way and may be detected in the methods disclosed herein and thus be used to determine its presence in an organism. From determining its nucleotide sequence, it may further allow determination of the genotype of the pathogen or microorganism and thereby the origin of the polynucleotide. This allows for identification of the specific microbial organism or pathogen or at least its genus which is responsible for the clinical condition of the subject and the disease may be identified.

The polynucleotide may also be "microRNA" (miRNA). MiRNAs are small, noncoding and highly conserved single-stranded RNA molecules which regulate gene expression by targeting messenger RNAs (mRNAs). A definition and further information can be found in Fu Y et al., 2011, Journal of Clinical Microbiology, 49:4246-4251. "Multiple small RNA" denotes short RNA molecules which may be detected by the methods disclosed herein.

A "polynucleotide indicative of a pathogen infection" or "indicative" in relation to polynucleotide means that the polynucleotide may be assigned to a specific pathogen or pathogen genus, e.g. or *i.e.* microbial genus or bacterial genus or microorganism or bacterium, or to a specific disease/infection which is caused by a pathogen. Thereby, also a pathogen infection, such as a microbial or bacterial infection, may be determined. The terms "determine" or "determining" are to be understood according to their general meaning in the field of medicine and medical diagnostics.

A "value indicative of the presence and/or amount of the polynucleotide" means that a concentration, an amount, ratio or any other value or feature may be determined which informs the practitioner on the presence/or amount of polynucleotide or pathogen in a sample of the bodily fluid.

The term "baseline value" as used herein, refers to a value that is used as a comparison value, e.g. to which a test result or assay value is compared. In practical terms, this may mean that an assay value (also referred to as a second value or simply as the value) is measured in a sample from a subject, and the result is compared to the baseline value. A value above the baseline may indicate a first likelihood of a diagnosis, and a value at about the baseline indicates a second likelihood of a diagnosis.

The first value or baseline value may be determined from a sample, e.g. referred to as the first sample or baseline sample of bodily fluid which is obtained before or prior to treatment of the subject with the anti-pathogenic agent.

The first value or baseline value may also be determined from a sample which is obtained before occurrence of the early response peak, e.g. after start of therapy but before the amount of polynucleotide is increased in response to the administration of the anti-pathogenic agent.

The first value or baseline value may also be determined from a sample which is obtained from a subject after or during treatment with a first anti-pathogenic agent but before treatment with a second anti-pathogenic agent, e.g. if the pathogen is not susceptible to the treatment with the first anti-pathogenic agent. In this embodiment, the second value or assay value is determined in a sample obtained from the subject after administration of the second anti-pathogenic agent.

A variety of methods may be used by the skilled artisan to arrive at a desired baseline value (also referred to here as a "first value"). In certain embodiments, the baseline value is determined from a sample of bodily fluid obtained from the same subject. In this embodiment, the change in amount of polynucleotide in samples of the same subject may be observed over time, and an increased amount of the polynucleotide provides an indication that the subject is infected with a pathogen and/or of worsening pathogen infection status in the subject.

In addition, or alternatively, the baseline value may be determined based on samples obtained from a population of subjects. Typically, the baseline value provides an acceptable level of specificity and sensitivity in separating the population into a first sub-population (e.g. having a pathogen infection) relative to the remaining second sub-population (e.g. not having a pathogen infection). As discussed herein, a typical baseline values separates this first and second population by one or more of the following measures of test accuracy:
- an odds ratio of at least about 2 or more, or about 0.5 or less; for example at least about 3 or more, or about 0.33 or less; or of at least about 4 or more, or about 0.25 or less; or of at least about 5 or more, or about 0.2 or less; or at least about 10 or more, or about 0.1 or less;
- at least 75% sensitivity, combined with at least 75% specificity;
- a ROC curve area of at least 0.6, e.g. at least 0.7, or at least 0.8, or at least 0.9, or at least 0.95; and/or
- a positive likelihood ratio (calculated as sensitivity/(1-specificity)) of at least 5, e.g. at least 10 or at least 20; or a negative likelihood ratio (calculated as (1-sensitivity)/specificity) of less than or equal to 0.3, e.g. less than or equal to 0.2 or less or equal to 0.1.

The term "about" in this context refers to +/- 5% of a given measurement.

The value or assay value or second value may be determined from a sample, e.g. referred to as the second sample, of the bodily fluid which is obtained during or after treatment of the subject with the anti-pathogenic agent and so forth. It is possible to administer to a subject another anti-pathogenic agent, another dose of anti-pathogenic agent or a different anti-pathogenic agent before obtaining additional samples, e.g. referred to as the third or more sample of the bodily fluid, in order to establish the presence of or promote a new raise of the polynucleotide concentration in the bodily fluid. For the single performance of the methods described herein it is generally appropriate to determine a first and a second value. However, in particular if monitoring is intended, more than a first and a second value can be determined from more than a first and a second sample of bodily fluid.

Both the first value (also referred herein as the baseline value) and second value (also referred herein as the assay value or simply as the value) may be selected from the group consisting of the parameters copy number of polynucleotide, concentration of polynucleotide, AUC (area under the curve) in a plot of concentration or copy number of polynucleotide in the sample against time, and peak height in relation to peak width of the early response peak. For example, the time for arriving at a certain percentage of a reference or baseline AUC, e.g. at least 30%, or at least 40%, or at least 50%, or at least 60%, or at least 70%, or at least 75%, or at least 80%, or at least 85 %, or at least 90%, or 100% may be characteristic for a subject having a pathogen infection or for a subject having no pathogen infection and/or for the effectiveness of treatment with the used anti-pathogenic agent. In another exemplary embodiment, the peak width in relation to the peak height of the early response peak may indicate a subject having a pathogen infection, a subject having no pathogen infection and/or the effectiveness of treatment with the anti-pathogenic agent as polynucleotides are released to the bodily fluid over a certain period of time. For example, a relatively steep and pronounced peak may indicate a high effectiveness of treatment with the used anti-pathogenic agent or antibiotic. In another exemplary embodiment, the time between start of peak increase above the baseline value of the early response peak to half maximum of the early response peak may indicate a subject having a pathogen infection, a subject having no pathogen infection and/or the effectiveness of treatment. For example, a relatively short period of time between start of peak increase above the baseline value of the early response peak to half maximum of the early response peak may indicate a high effectiveness of treatment with the used anti-pathogenic agent or antibiotic.

"Comparing" the second and the first or any other following value is to be understood according to its general meaning in the fields of diagnostics and mathematics. In typical embodiments, if the second value is higher than the first value, this indicates a pathogen infection, the presence and/or genotype of a pathogen due to the administration of the anti-pathogenic agent or treatment of the patient with the anti-pathogenic agent. Thereby, the pathogen infection, the presence and/or genotype of the pathogen may be determined.

The term "diagnosis" as used herein, refers to methods by which the skilled person can estimate and/or determine whether or not a patient is suffering from a given disease or condition, e.g. a pathogenic infection. The skilled person often makes a diagnosis on the basis of one or more diagnostic indicators, e.g. a marker, the presence, absence, amount or change in amount of which is indicative of the presence, severity or absence of the pathogenic infection. The term "diagnosis" does not refer to the ability to determine the presence or absence of a pathogenic infection with 100% accuracy, or even that a given course or outcome is more likely to occur than not. Instead, the skilled person will understand that the term "diagnosis" refers to an increased probability that a certain pathogenic infection is present in the subject.

"Monitoring" denotes the observation of a disease development of a patient. For monitoring, the treatment may start with the method described herein for determining or diagnosing a pathogen infection, the presence and/or amount of a polynucleotide and/or the presence and/or genotype of a pathogen and continue with the method for monitoring as also described herein. In between, the anti-pathogenic agent may be changed, combined with one or more other anti-pathogenic agents or the doses may be changed. Thereby, the efficiency of the anti-pathogenic agent with respect to the specific pathogen may be observed and also the clinical picture of the patient.

The term "monitoring the presence and/or amount of a polynucleotide" refers to observing the presence and/or amount of a polynucleotide over time. In particular, a plurality of values, e.g. a second, third, fourth, etc. value indicative of the presence and/or amount of the polynucleotide may be determined in corresponding samples of bodily fluid, as described herein.

As used herein, a "plurality" refers to at least 2, e.g. to at least 3, or at least 5, or at least 10, or at least 15, or at least 20. In specific embodiments, a plurality is a large number, e.g. at least 100.

The term "monitoring a pathogenic infection" refers to observing a patient diagnosed with the pathogen infection for changes in pathogen infection status, as measured by a change in presence and/or amount of a polynucleotide indicative of the pathogen infection. Based on the observations, the skilled artisan can initiate or alter treatment of a subject, e.g. in advance of the subject exhibiting outward clinical signs or deteriorating pathogen infection status.

An "anti-pathogenic agent" is a chemical substance which has anti-pathogenic effect, *i.e.* leads to the death of the pathogen or to growth inhibition of the pathogen. An example of an anti-pathogenic agent is an antibiotic. "Antibiotic" denotes a chemical substance which has antimicrobial or antibacterial effect in that the growth of a microorganism, such as a bacterium, is inhibited (bacteriostatic effect), and/or the microorganism is killed (bactericidal effect) which leads to the death and subsequent degradation of the microbial cell. Thereby, microbial/bacterial genetic material in the form of polynucleotides is released from the cell. The term "antibiotic" also includes antibiotic analogs which may be semi-synthetic or synthetic and which are chemical substances that kill bacteria. These analogs may be derived from echinacea, garlic, goldenseal, myrrh, or others, or comprise e.g. colloidal silver or cathelicidins. An antibiotic may also be of natural origin ("natural antibiotic") which is generally produced by another microorganism such as fungi and well understood by a person skilled in the art. An antibiotic is "effective" against a microorganism or bacterium if it has bacteriostatic and/or bactericidal effect.

"Bodily fluid" is to be understood according to its meaning in the field of medicine and denotes any possible fluid which may be obtained from a subject, such as a human or animal. "Obtaining" or "to obtain" are to be understood according to their general meaning in the field of medicine and medical diagnostics and denote the retrieval or taking of a sample of bodily fluid from a subject, such as taking of a blood sample from a subject or a urinary or sputum or any other kind of bodily fluid probe of a subject depending on the bodily fluid which is to be obtained. Typical bodily fluids may be tears, lacrimal fluid, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, liquid stool, fluid harvested from a site of an immune response, fluid harvested from a pooled collection site, bronchoalveolar lavage, urine, or blood sample. The blood sample may be a whole blood sample, plasma or serum sample or a blood sample unable to coagulate. In other embodiments, the sample of a bodily fluid may be a sputum sample. In addition, one of skill in the art would realize that a sample may be more readily analyzed following a fractionation or purification procedure, for example separation of whole blood into serum or plasma components.

In certain embodiments, a bodily fluid of a subject is chosen as the sample type for the methods described herein because this bodily fluid typically does not contain the pathogen. For example, the bodily fluid is a bodily fluid which does not contain the pathogen.

In certain embodiments, such a sample may be obtained for the purpose of diagnosis, prognosis or evaluation of a subject. For example, such a sample may be obtained for the purpose of determining the outcome of an ongoing condition or the effect of a treatment regimen on a condition.

Prior or before treatment of the subject with the anti-pathogenic agent, a first sample of a bodily fluid is obtained from the subject. During or after treatment or after administration of the anti-pathogenic agent to the subject, a second sample of a bodily fluid is obtained from the subject. Hence, "first sample" refers to an amount or volume of a bodily fluid which may be obtained before treating the subject with or before administering to the subject an anti-pathogenic agent and "second sample" refers to an amount or volume of a bodily fluid which may be obtained after treating the subject with an anti-pathogenic agent or after having administered to the subject an anti-pathogenic agent. Typically, the first sample of a bodily fluid and the second sample of a bodily fluid are of the same kind, e.g. in both cases the bodily fluid is urine, or blood or any other bodily fluid mentioned herein. The amount or volume of the first and the second or even more samples of the bodily fluid may be chosen so that enough material for subsequent performing of the method disclosed herein is obtained. Optionally, 0.1 mL, 0.5 mL, 1 mL, 2 mL, 3 mL, 5 mL, 6 mL, 10 mL or 15 mL or even more may be obtained. The amount may also depend on the kind of bodily fluid, e.g. in case of urine in general more volume of bodily fluid may easily be obtained than of blood.

A "subject" or "patient" may be a human or non-human, e.g. animal organism. Thus, the methods described herein may be applicable to both human and veterinary pathogen infections. Further, while a subject is typically a living organism, the methods described herein may be used in post-mortem analysis as well. Typical subjects are "patients", e.g. living humans that are receiving medical care or treatment for a disease or condition. This includes persons with no defined illness or being investigated for signs of pathology.

The "early response peak" denotes an increase in the polynucleotide or oligonucleotide concentration in a bodily fluid in response to the administration of an anti-pathogenic agent or antibiotic to the subject from which the first and second or even more samples of bodily fluid were obtained. The early response peak may give information about the amount and/or type of pathogen or microorganism present in the subject and/or about the effectiveness of the anti-pathogenic agent or antibiotic towards the respective pathogen in the subject. In case of two or more pathogens present in the subject, it may even be the case that two early response peaks appear in overlapping manner or timely separated. The presence of separated or partly or fully overlapping peaks may give information about the amount of the two or more pathogens in the subject or about the effectiveness of the anti-pathogenic agent or antibiotic towards both or more pathogens. From the early response peak one may also derive information on the estimated length of the required treatment and/or dose to be administered.

The terms "administration" or "administering" refers to a method of giving an amount or a dosage of an anti-pathogenic agent or antibiotic to a subject or a patient. The terms "administering" or "administration" include single administration or repeated administration of an anti-pathogenic agent to a subject. Repeated administration includes administration e.g. once or twice a day over a period of several days, e.g. 6 to 20 days such as 7, 8, 9, 10, 11, 12, 13 14, 15, 16, 17, 18, or 19 days. In special embodiments, repeated administration includes administration over a period of up to three months. The term "sample obtained after administration" refers to samples obtained after treatment initiation start of therapy with the anti-pathogenic agent, e.g. after a single or several, e.g. 2, 3, 4, 5, 6, 7, 8, 9 or 10 administrations of the anti-pathogenic agent and also includes samples which have been obtained during therapy with the anti-pathogenic agent. The term "sample obtained before administration" refers to samples which have been obtained before start of therapy with the anti-pathogenic agent, e.g. the first administration of the anti-pathogenic agent.

The terms "treat", "treatment", or "treating" or "treatment of the subject with an anti-pathogenic agent" or the like denotes administering an anti-pathogenic agent, such as an antibiotic, to a subject. Administering the anti-pathogenic agent may have the effect or purpose of increasing the amount of polynucleotides in a bodily fluid for better and more reliable detection of said polynucleotide in the bodily fluid. A "subject treated with an anti-pathogenic agent" includes a subject that was or still is being treated with an anti-pathogenic agent according to standard dosage regimens and/or treatment schedules. Such a treatment may include a single or repeated administration of the anti-pathogenic agent to the subject.

"Isolating" a polynucleotide from a sample of a bodily fluid denotes that the polynucleotide is separated from other components of the bodily fluid such as cells, cell debris and other components in solution so that e.g. an amplification of the polynucleotides can be performed. "Amplification" of a polynucleotide denotes in the broadest sense the increase of the copy number of a polynucleotide. "Detecting" the polynucleotide denotes that the polynucleotide is specifically measured or determined in a sample so that the presence of a pathogen and/or a pathogen infection and/or the genotype of the pathogen may be identified.

A pathogen may be a microorganism, optionally a bacterium, yeast, fungi, single-celled plants or other eukaryotes and prokaryotes. Preferably, the pathogen is a bacterium.

Exemplary pathogen may be of the genus *Mycobacterium, Helicobacter, Bacillus, Leishmania, Chlamydia, Neisseria, Streptococcus, Pseudomonas, Escherichia, Klebsiella, Enterobacter, Proteus, Enterobacteria, Staphylococcus, Cryptococcus, Histoplasma, Aspergillus, Schistosoma, Legionella* and/or *Plasmodium* or other pathogens which may cause a disease in human or animal subjects. The method disclosed herein is also suitable to detect polynucleotides of one or more pathogens within a bodily fluid of a subject. Thereby, the method is also suitable to detect more than one infection or disease of the subject. In the subject, one or more pathogens or bacteria may be present in any combination of pathogens and bacteria.

Typical examples of pathogens are *Mycobacterium tuberculosis* (MTB), *Mycobacterium bovis, Mycobacterium leprae, Mycobacterium africanum*, *Helicobacter pylori, Bacillus anthracis, Chlamydia trachomatis, Neisseria gonorrhoeae, Neisseria meningitidis, Streptococcus pneumonia, Chlamydia pneumonia, Pseudomonas aeruginosa, Escherichia coli (E. coli), Klebsiella species (spec.), Enterobacter spec., Proteus spec.* and other *Enterobacteria, Staphylococcus aureus, Cryptococcus neoformans, Histoplasma capsulatum, Aspergillus spp (species pluralis=spp), Schistosoma mansoni, Schistosoma haemotobium*, *Legionella pneumophila*, *Plasmodium spec.*, and combinations thereof.

Preferred pathogens are bacteria, such as *Mycobacterium tuberculosis* (MTB), *Mycobacterium africanum, Mycobacterium bovis, Mycobacterium leprae, Helicobacter pylori, Bacillus anthracis, Chlamydia trachomatis, Neisseria gonorrhoeae, Neisseria meningitidis, Streptococcus pneumonia, Chlamydia pneumonia, Pseudomonas aeruginosa, Escherichia coli (E. coli), Klebsiella species (spec.)*, *Enterobacter spec., Proteus spec.* and other *Enterobacteria* and *Staphylococcus aureus,* and combinations thereof. A particular preferred pathogen is *Mycobacterium tuberculosis.*

The disease, pathogen infection or clinical picture in which the method described herein may help to identify and determine the pathogen responsible for the disease or clinical picture or the presence of such pathogen may be selected from the group consisting of tuberculosis, pulmonary tuberculosis, paediatric, extrapulmonary or HIV-associated tuberculosis, leprosy, chronic gastritis, gastric ulcers, peptic ulcers, anthrax, gonorrhoea, orchitis, conjunctivitis, pharyngitis, proctitis, urethritis, prostatitis, bacterial meningitis, infertility, meningococcal septicaemia, community acquired pneumonia (CAP), sepsis, meningitis, urinary tract infections, pyelonephritis, pneumonia, pyelonephritis, nosocomial or ventilator-associated pneumonia, lung infections, optionally in immune-compromised (HIV=human immunodeficiency virus) patients, histoplasmosis, disseminated mycoses, schistosomiasis, malaria, and combinations thereof. Optionally the disease or pathogen infection is selected from tuberculosis, sepsis, meningitis, urinary tract infections and lung infections.

A subject may not only suffer from a single pathogen infection or disease but also from one or more, such as 2, 3, 4 or five pathogen infections or diseases at the same time. Beside this, the subject may additionally suffer from different, one or more, viral infections, different cancers and/or genetic diseases. This may make it even more difficult to identify a pathogen infection or disease simply from the symptoms of the patient. Moreover, a treatment with a suboptimal anti-pathogenic agent or antibiotic may be highly critical to the subject. In particular, in these cases of combined infections and diseases the methods disclosed herein are useful to determine the specific pathogen being responsible for the infection via detecting a polynucleotide being derived from the pathogen and thereby identifying the pathogen infection.

Exemplary bodily fluids are urine, bronchoalveolar lavage, plasma, serum, lymphatic fluid, lacrimal fluid, sputum, tears, saliva, nasal fluid, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, liquid stool, fluid harvested from a site of an immune response, fluid harvested from a pooled collection site, bronchial lavage, blood, such as whole blood, plasma, serum or blood unable to coagulate, and other fluids known to a person skilled in the art. Optionally, the bodily fluid may be blood, plasma, serum or urine. A person skilled in the art further well understands that blood comprises serum and plasma. Plasma is generally regarded as the cell-free liquid of blood and still contains fibrinogen. Serum is the liquid part of the blood which remains after removal of fibrinogen. Additional information on bodily fluids can be taken from "Graffs Textbook of Urinalysis and Body Fluids" by L. Mundt and K. Shanahan (2010, Lippincott Williams & Wilkins).

The samples of the bodily fluids which comprise the polynucleotides may be obtained by any method regarded suitable by a person skilled in the art. Exemplary methods include e.g. urinating, removal or extraction of blood and spitting and coughing for sputum and bronchoalveolar lavage. Serum and plasma samples are subsequently obtained from blood by standard methods known in the art. Further information thereon can be taken from "Guide to the Preparation, Use and Quality Assurance of Blood Components" by the Council of Europe (2007, Council of Europe).

Urine as bodily fluid is particularly advantageous as urine may be obtained from the subject in a non-invasive way. Moreover, HIV is not transferred via urine so that infection risk is low for diagnosing staff in case of HIV-coinfected subjects. Although it is believed that only about 5% of polynucleotides escape degradation in blood and kidney, the early response peak which may be detected in urine may be equal or even more pronounced in height than the peak which can be measured in blood or plasma. Without intention to be bound by a particular theory, it is believed that a concentration of the polynucleotides in urine may be responsible for this higher measureable amount of polynucleotides in urine. Moreover, polynucleotides may have a lower half-life in circulating blood/plasma due to nucleases. In some embodiments, the bodily fluid is to be chosen so that the risk of infection of the analyst is reduced to a minimum on the one hand, and the accuracy of the detection of the polynucleotide and thus of the pathogen infection is at a maximum on the other hand.

Examples of anti-pathogenic agents are isoniazid (INH), rifampin/rifampicin (RIF), pyrazinamide (PZA), ethambutol (EMB or ETB), streptomycin (SM), prothionamid (PTH), dapsone, clofazimine, clarithromycin, amoxicillin, fluoroquinolones, e.g. ciprofloxacin, doxycycline, erythromycin, vancomycin, silver nitrate, penicillins, tetracyclines, cefotaxime, ceftriaxone, aminopenicillins, cephalosporines, macrolides, tetracycline, azithromycin, acylaminopenicillins, aminoglycosides, carbapenems, flucloxacillin, teicoplanin, amphotericin B, fluconazol, azoles, posacanazol, echinocandines, e.g. caspofungin and mycamine, praziquantel, oxamniquin, erythromycin, quinine, mefloquine, resoquine, gentamicin, clindamycin, and combinations thereof. Preferred anti-pathogenic agents are antibiotics being effective against *Mycobacterium tuberculosis* such as rifampin/rifampicin, pyrazinamide, ethambutol and streptomycin and any combination thereof. Further information on anti-pathogenic agents can be found in "Colloquium on Virulence and Defense in Host-Pathogen Interactions: Common Features between Plants and Animals" by N.T. Keen (2001, National Academies Press).

Further information on antibiotics can be found in "Antibiotics:
An Overview" by K. Drlica and D. Perlin (2011, Pearson Education) and "Peptide Antibiotics: Discovery Modes of Action and Applications" by C.J. Dutton et al. (2001, CRC Press). Information on semi-synthetic antibiotics may be found in "Structure-activity relationships among the semisynthetic antibiotics" by D. Perlman (Academic Press, 1977).

The antibiotic may be a broadband antibiotic, such as gentamicin and clindamycin. The use of a broadband antibiotic may be useful in cases where the symptoms of the patient are such vague that no clear or reasonable estimation of the responsible bacteria can be made. Also here, the early response peak may be used to genotype the pathogen, in particular the bacterium, and switch to a treatment with a specifically suitable antibiotic, subsequently.

The anti-pathogenic agent, antibiotic or antibiotic analog may be administered in any suitable way depending on its formulation, the condition of the patient or subject and the preference of the physician. The way of administration can vary depending on various factors, *e.g.* the components of the composition comprising the anti-pathogenic agent, the site of the potential or actual pathogen infection, the pathogen involved and the severity of the actual pathogen infection. Examples of way to administer the antibiotic are intravenous, pulmonary by inhalation of an aerosol through mouth or throat, oral in form of a tablet or liquid or else.

Dosage of the anti-pathogenic agent is to be chosen according to the severity of the symptoms of the patient, the overall constitution of the patient and the anti-pathogenic agent or antibiotic to be administered. Such decision is in general to be taken by the physician in charge. The administration can be done only once or repeatedly depending on before mentioned factors. Optionally, the amount of anti-pathogenic agent or antibiotic can be at least 20 mg, 25 mg, 30 mg, 35 mg, 40 mg, 50 mg, 60 mg, 70 mg, 80 mg, 90 mg, 100 mg, 1 10 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 460 mg, 470 mg, 480 mg, 490 mg, 500 mg, 510 mg, 520 mg, 530 mg, 540 mg, 550 mg, 560 mg, 570 mg, 580 mg, 590 mg, 600 mg, 610 mg, 620 mg, 630 mg, 640 mg, 650 mg, 660 mg, 670 mg, 680 mg, 690 mg, 700 mg, 710 mg, 720 mg, 730 mg, 740 mg, 750 mg, 760 mg, 770 mg, 780 mg, 790 mg, 800 mg, 900 mg, or 1 g per event of administration.

An exemplary dosage regimen in case of a patient being suspected of tuberculosis may be a combination treatment with isoniazid and/or rifampicin and/or pyrazinamide and/or ethambutol for 2 months. After possible development of a resistance of the mycobacterial strain, the therapy is continued with isoniazid and rifampicin for at least 4 month, optionally with another combination of antibiotics depending on the monitoring results. Treatment may be necessary for about 6 month in case of pulmonary manifestation. In particular, the exemplary dosage regimen may comprise administration of pyrazinamide (Pyrofat, Junek Europ V. GmbH Zn or pyrazinamide generics, Riemser Arzneimittel AG, dose 35 mg/kg body weight, 2 g, p.o.), ethambutol (EMB-Fatol, Riemser Arzneimittel AG, or EMB-Hefa, Sanav.Werniger, or myambutol, Emra-Med Arzneimittel GmbH, dose 25 mg/kg body weight p.o., rifampicin (Eremfat, Riemser Arzneimittel AG, or Rifa, Riemser Arzneimittel AG, dose 10 mg/kg body weight, 600 mg p.o. and isoniazid (isoniazid generics, Pfizer Limited, or Macleods Pharmaceuticals Pvt Ltd,or Cyper Pharma, dose 5 mg/kg body weight, 300 mg p.o. Optionally, the anti-pathogenic agent in the method may be administered or the subject is treated with the anti-pathogenic agent in the following way: with isoniazid and/or rifampicin and/or pyrazinamide and/or ethambutol. Optionally, the anti-pathogenic agent may be isoniazid in a dose of 5 mg/kg body weight, 300mg p.o.; and/or rifampicin in a dose of 10 mg/kg body weight, 600 mg p.o.; and/or pyrazinamide in a dose 35 mg/kg body weight, 2 g, p.o.; and/or ethambutol in a dose 25 mg/kg body weight p.o.

The frequency of administration can be repeated depending on to the severity of the symptoms of the patient, the overall constitution of the patient and the anti-pathogenic agent or antibiotic to be administered. The frequency can be *e.g.* every hour, every second hour, every 3, 4, 5, 6, 7, 8, 10, 11, 12, 13, 14, 15, 16, 17, 18, 19, 20, 21, 22, 23 hours, every day, once a day, twice a day, every 3 hours, every 6 hours, every 2, 3, 7, 10, 14 days, every month, every 2, 3, 4, 6, 9, 12 month, every second or third, fifth or tenth year.

Of course, depending on the symptoms of the patient also a combined administration of one or more anti-pathogenic agents/antibiotics may be done. The amount of anti-pathogenic agent/antibiotic may be the same or different and be chosen by the physician in charge.

The following Table 1 gives an overview of exemplary combinations of pathogen, disease or pathogen infection, bodily fluid and anti-pathogenic agent.

**Table 1: Overview of exemplary microorganisms related diseases, bodily fluids in which a polynucleotide of the mentioned microorganism may appear after administration of a subject with one or more of the mentioned antibiotic.**

| **Pathogen** | **Disease/Relevance** | **Bodily fluid** | **Anti-pathogenic agent** |
|---|---|---|---|
| *Mycobacterium tuberculosis, Mycobacterium bovis, Mycobacterium africanum Mycobacterium canettii, Mycobacterium microti* | Tuberculosis, pulmonary tuberculosis, paediatric, extrapulmonary, HIV-associated tuberculosis, exclusion urogenital tuberculosis | Blood, urine, bronchoalveolar lavage | Isoniazid (INH), Rifampin/ Rifampicin (RIF), Pyrazinamide (PZA), Ethambutol (EMB), streptomycin (SM) |
| *Mycobacterium leprae* | leprosy | Blood, urine | Dapsone, Rifampicin and Clofazimine |
| *Helicobacter pylori* | chronic gastritis, gastric ulcers, peptic ulcers | Blood, urine | Clarithromycin, Amoxicillin |
| *Bacillus anthracis* | Anthrax | Blood, urine | Fluoroquinolones (e.g. Ciprofloxacin), Doxycycline, Erythromycin, Vancomycin, Penicillin |
| *Neisseria gonorrhoeae, Neisseria meningitidis* | Gonorrhoea, orchitis, conjunctivitis, pharyngitis, proctitis, urethritis, prostatitis, bacterial meningitis, infertility, meningococcal septicaemia | Blood, urine | Silver nitrate, Penicillins, tetracyclines, Cefotaxime, Ceftriaxone |
| *Streptococcus pneumoniae* | CAP - community acquired pneumonia, sepsis, meningitis | Blood, urine | Penicillins, Aminopenicillins, Cephalosporines, Rifampicin, Vancomycin |
| *Chlamydia trachomatis, Chlamydia pneumonia* | CAP - community acquired pneumonia | Urine | Macrolides, Tetracycline, Doxycyclin, Azithromycin |
| *Pseudomonas aeruginosa* | Sepsis, Urinary tract infections, pyelonephritis, pneumonia | Blood, urine bronchoalveolar lavage | Acylaminopenicillins, Cephalosporins, Fluoroquinolones, Aminoglycosides, Carbapenems |
| *E. coli, Klebsiella spec., Enterobacter spec., Proteus spec. and other enterobacteria* | Sepsis, Urinary tract infections, and pyelonephritis, nosocomial or ventilator-associated pneumonia | Blood, urine bronchoalveolar lavage | Amino-/ Acylaminopenicillins, Cephalosporins, Fluoroquinolones, Aminoglycosides, Carbapenems |
| *Staphylococcus aureus* | Sepsis, pneumonia | Blood, bronchoalveolar lavage, urine | Flucloxacillin, Cephalosporines, Rifampicin, Vancomycin, Teicoplanin |
| *Cryptococcus neoformans* | Meningitis and lung infections in immunocompro-mised (HIV) patients | Plasma, blood, urine | Amphotericin B |
| *Histoplasma capsulatum* | Histoplasmosis | Plasma, blood, urine | Amphotericin B and Fluconazol |
| *Aspergillus spp* | Disseminated Mycoses | Serum, urine, bronchoalveolar lavage | Azoles, Posacanazol, Amphotericin B, Echinocandines (Caspofungin, Mycamine) |
| *Schistosoma mansoni, Schistosoma haemotobium* | Schistosomiasis | Urine, blood | Praziquantel, Oxamniquin |
| *Legionella pneumophila* | CAP - community acquired pneumonia | Urine | Erythromycin, Rifampicin, Fluoroquinolones |
| *Plasmodium spec.* | Malaria | Blood, urine | Quinine, Mefloquine, Resoquine and others |

In an exemplary embodiment, the pathogen is or the polynucleotide is derived from *Mycobacterium tuberculosis, Mycobacterium africanum, Mycobacterium canettii, Mycobacterium microti* or *Mycobacterium bovis,* the pathogen infection or disease is selected from tuberculosis, pulmonary tuberculosis, paediatric, extrapulmonary and HIV-associated tuberculosis, the bodily fluid is blood, urine or bronchoalveolar lavage, and the anti-pathogenic agent is selected from the group consisting of isoniazid (INH), rifampin/rifampicin (RIF), pyrazinamide (PZA), ethambutol (EMB), streptomycin (SM) and combinations thereof. In this embodiment, the bodily fluid is typically blood or urine, e.g. urine. Specifically, the pathogen is or the polynucleotide is derived from *Mycobacterium tuberculosis,* the pathogen infection or disease is tuberculosis, the bodily fluid is blood or urine, e.g. urine, and the anti-pathogenic agent is selected from the group consisting of isoniazid (INH), rifampin/rifampicin (RIF), pyrazinamide (PZA), ethambutol (EMB), streptomycin (SM) and combinations thereof.

In another exemplary embodiment, the pathogen is or the polynucleotide is derived from *Mycobacterium leprae,* the pathogen infection or disease is leprosy, the bodily fluid is blood or urine, and the anti-pathogenic agent is selected from the group consisting of dapsone, rifampicin, clofazimine and combinations thereof.

In another exemplary embodiment, the pathogen is or the polynucleotide is derived from *Helicobacter pylori,* the pathogen infection or disease is selected from chronic gastritis, gastric ulcers and peptic ulcers, the bodily fluid is blood or urine, and the anti-pathogenic agent is clarithromycin and/or amoxicillin.

In another exemplary embodiment, the pathogen is or the polynucleotide is derived from *Bacillus anthracis,* the pathogen infection or disease is anthrax, the bodily fluid is blood or urine, and the anti-pathogenic agent is selected from the group consisting of fluoroquinolones (e.g. ciprofloxacin), doxycycline, erythromycin, vancomycin, penicillin, and combinations thereof. In this embodiment, the bodily fluid is typically urine.

In another exemplary embodiment, the pathogen is or the polynucleotide is derived from *Neisseria gonorrhoeae* or *Neisseria meningitidis,* the pathogen infection or disease is selected from the group consisting of gonorrhoea, orchitis, conjunctivitis, pharyngitis, proctitis, urethritis, prostatitis, bacterial meningitis, infertility, meningococcal septicaemia, and combinations thereof, the bodily fluid is blood or urine, and the anti-pathogenic agent is selected from the group consisting of silver nitrate, penicillins, tetracyclines, cefotaxime, ceftriaxone, and combinations thereof.

In another exemplary embodiment, the pathogen is or the polynucleotide is derived from *Streptococcus pneumoniae,* the pathogen infection or disease is selected from the group consisting of community acquired pneumonia, sepsis, meningitis, and combinations thereof, the bodily fluid is blood or urine, and the anti-pathogenic agent is selected from the group consisting of penicillins, aminopenicillins, cephalosporines, rifampicin, vancomycin, and combinations thereof. In this embodiment, the bodily fluid is typically urine.

In another exemplary embodiment, the pathogen is or the polynucleotide is derived from *Chlamydia trachomatis* and *Chlamydia pneumonia,* the pathogen infection or disease is selected from the group consisting of community acquired pneumonia, pneumonia, and combinations thereof, the bodily fluid is urine, and the anti-pathogenic agent is selected from the group consisting of macrolides, tetracycline, doxycycline, azithromycin, and combinations thereof.

In another exemplary embodiment, the pathogen is or the polynucleotide is derived from *Pseudomonas aeruginosa,* the pathogen infection or disease is selected from the group consisting of sepsis, urinary tract infections, pyelonephritis, pneumonia, and combinations thereof, the bodily fluid is blood, urine or bronchoalveolar lavage, and the anti-pathogenic agent is selected from the group consisting of acylaminopenicillins, cephalosporines, fluoroquinolones, aminoglycosides, carbapenems, and combinations thereof. In this embodiment, the bodily fluid is typically bronchoalveolar lavage.

In another exemplary embodiment, the pathogen is or the polynucleotide is derived from a pathogen being selected from the group consisting of *E. coli, Klebsiella spec., Enterobacter spec., Proteus spec.* other *Enterobacteria,* and combinations thereof, the pathogen infection or disease is selected from the group consisting of sepsis, urinary tract infections, pyelonephritis, nosocomial or ventilator-associated pneumonia, and combinations thereof, the bodily fluid is blood, urine or bronchoalveolar lavage, and the anti-pathogenic agent is selected from the group consisting of amino-/acylaminopenicillins, cephalosporines, fluoroquinolones, aminoglycosides, carbapenems, and combinations thereof.

In another exemplary embodiment, the pathogen is or the polynucleotide is derived from *Staphylococcus aureus,* the pathogen infection or disease is sepsis and/or pneumonia, the bodily fluid is blood, urine or bronchoalveolar lavage, and the anti-pathogenic agent is selected from the group consisting of flucloxacillin, cephalosporines, rifampicin, vancomycin, teicoplanin, and combinations thereof. In this embodiment, the bodily fluid is typically urine.

In another exemplary embodiment, the pathogen is or the polynucleotide is derived from *Cryptococcus neoformans*, the pathogen infection or disease is selected from the group consisting of meningitis and lung infections in immune-compromised (such as HIV) patients, and combinations thereof, the bodily fluid is blood, plasma or urine, and the anti-pathogenic agent is amphotericin B. In this embodiment, the bodily fluid is typically plasma.

In another exemplary embodiment, the pathogen is or the polynucleotide is derived from *Histoplasma capsulatum*, the pathogen infection or disease is histoplasmosis, the bodily fluid is blood, urine or plasma, and the anti-pathogenic agent is amphotericin B and/or fluconazol. In this embodiment, the bodily fluid is typically blood or plasma.

In another exemplary embodiment, the pathogen is or the polynucleotide is derived from *Aspergillus spp*, the pathogen infection or disease is disseminated mycoses, the bodily fluid is serum, urine, blood, plasma or bronchoalveolar lavage, and the anti-pathogenic agent is selected from the group consisting of azoles, posacanazol, amphotericin B, echinocandines, caspofungin, mycamine, and combinations thereof.

In another exemplary embodiment, the pathogen is or the polynucleotide is derived from *Schistosoma manson* and/or *Schistosoma haemotobium*, the pathogen infection or disease is schistosomiasis, the bodily fluid is urine or blood, and the anti-pathogenic agent is praziquantel and/or oxamniquin. In this embodiment, the bodily fluid is typically blood.

In another exemplary embodiment, the pathogen is or the polynucleotide is derived from *Legionella pneumophila*, the pathogen infection or disease is community acquired pneumonia, the bodily fluid is urine, and the anti-pathogenic agent is selected from the group consisting of erythromycin, rifampicin, fluoroquinolones, and combinations thereof. In this embodiment, the bodily fluid is typically serum.

In another exemplary embodiment, the pathogen is or the polynucleotide is derived from *Plasmodium spec.*, the pathogen infection or disease is malaria, the bodily fluid is urine or blood, and the anti-pathogenic agent is selected from the group consisting of quinine, mefloquine, resoquine, and combinations thereof. In this embodiment, the bodily fluid is typically urine.

For example, the bodily fluid, *i.e.* a sample of the bodily fluid, such as a first sample and/or a second sample, of a patient having symptoms of a disease which may or may not be clearly assigned to a specific disease can be tested for the presence of a polynucleotide. A first value determined from a first sample of a bodily fluid can be used as zero or starting or baseline value. Second, the antibiotic is administered and third a second sample of a bodily fluid is tested for the presence of the polynucleotide. After administration, e.g. immediately after administration, e.g. after 1 min, after 2 min, 3 min, 5, min, 10 min, 15 min, 20 min, 30 min, 45 min, 1 h, 1.5 h, 2 h, 3 h, 4 h, 5 h, 6 h, 8 h, 10 h, 12 h, 14 h, 16 h, 20 h, 24 h, after 1 day 2 days, 3 days, 4 days, 5 days, 6 days, 7 days, 8 days, 9 days or 10 days, in general, from 1 day after administration to 10 days after administration, or from 2 days to 8 days, or 3 days to 6 days or about 5 days the early response peak is detectable, *i.e.* an increase in the copy number of polynucleotide in the bodily fluid.

From the difference between the starting or first value and the second value which may represent the early response peak, information may be given e.g. about the effectiveness of the antibiotic, the type of pathogen/bacterium and optionally also about the disease or infection itself. The early response peak reflected in the second value may be detectable or can be determined by determining/detecting the polynucleotide in the second sample of a bodily fluid after 1 day to 20 days or 1 day to 10 days, optionally after 2 days to 8 days, or 3 days to 6 days, or about 4 to 6 days after administering the anti-pathogenic agent or antibiotic to the subject by any suitable way of administering and in any amount which is considered suitable by the physician in charge.

Hence, treating a subject with the anti-pathogenic agent/antibiotic or administering to the subject the anti-pathogenic agent/antibiotic is generally done 1 h, 1.5 h, 2 h, 3 h, 4 h, 5 h, 6 h, 8 h, 10 h, 12 h, 14 h, 16 h, 20 h or 1 day to 10 days prior to obtaining a second sample of the bodily fluid and/or isolating the polynucleotide from the second sample of the bodily fluid, optionally 2 to 8 days, 3 to 6 days, or about 4 to 6 days prior to obtaining and/or isolating the polynucleotide from the second sample of the bodily fluid. The isolating of the polynucleotide is performed timely after the step of obtaining the first sample of a bodily fluid from the subject. In some embodiments, the time between isolating the polynucleotide from the first sample of the bodily fluid and obtaining the second sample of the bodily fluid from the subject is as short as possible. Nevertheless, transportation, preparation of plasma and serum from blood and other preparatory steps have to be considered. In some embodiments, the first and second or more samples may be stored between obtaining the first or more sample of a bodily fluid and isolating the polynucleotide from the first or more sample of bodily fluid, *e.g.* by freezing or cooling the bodily fluid probe, *i.e.* the first or more sample of the bodily fluid. However, also in view of the disease and symptoms of the patient, quick analysis of the polynucleotides in the bodily fluids, in particular in the first sample of a bodily fluid, may be considered to be the typical way of performing the method. Nevertheless, the method disclosed herein may also be performed with first samples of bodily fluids which have been stored for a longer period of time.

The methods described herein or the test of the bodily fluid samples may then be repeated as long as no pathogen polynucleotide can be detected anymore. This test may be performed as often as it is regarded necessary by the medical practitioner, *e.g.* every hour, every 6 hours, every day, every 2, 3, 4, 5, 6 days, every week or every second week, every month, every three or six month, or every year or every two years. Hence, the methods disclosed herein are also suitable to monitor a patient's clinical picture over a longer period of time, even over years. In this case, more than a first and a second sample of a bodily fluid have to be taken.

The polynucleotide disclosed herein can be DNA, RNA, PNA or similar. The polynucleotide disclosed herein may be at least 10 bp in length or from 10 bp to about 2500 bp in length, preferably from 15 bp to about 1000 bp in length, more preferably, from 20 bp to about 800 bp in length, most preferably from 20 bp to about 500 bp, or from 30 bp to 300 bp in length.

The concentration of polynucleotide in the first and the second or even more samples of bodily fluid may be considerably low for detection purposes. About 1 copy per mL bodily fluid sample, or *e.g.* 5 to 10 copies per mL urine or blood, plasma or serum are still possible to detect. Hence, the concentration or amount before administration of the anti-pathogenic agent may be below this concentration of 10 copies per mL urine and may be or is increased by the administration of the anti-pathogenic agent which leads to an increase in the bodily fluids and thus in the samples. Preferably, the administration of the anti-pathogenic agent leads to a copy number per mL bodily fluid of 10, 20, 30, 40, 50, 60, 70, 80, 90, 100, 125, 150, 175, 200, 225, 250, 275, 300, 400, 500, 600, 700, 800, 900, 1000 or even more copies per mL bodily fluid.

The method step of isolating polynucleotides from the samples of bodily fluid may be performed according to standard procedures and techniques well known to a person skilled in the art. There exist a number of different techniques and kits which may be employed for this purpose and which may be purchased e.g. from Qiagen (Hilden, Germany), such as the PAXgene Blood DNA Kit, QIAamp DNA (Mini) Kit, RNeasy minikit or similar. Other useful kits in this respect can be purchased from Life Technologies (formerly known as Invitrogen, Carlsbad, CA, USA; *e.g.* TRIzol kit) and Promega (Mannheim, Germany; Guanidine Wizard Resin (GITC/WR) method). Generally, the kits are to be used according to the manufacturer's protocol.

The isolation can also be performed *e.g.* by precipitation or using a solid adsorbent material. Another example of polynucleotide isolation is the Triton/Heat/Phenol protocol (THP) (Xue et al., 2009, Clinica Chimica Acta, 404:100-104). One example of a method step of isolating polynucleotides from urine comprises separating cells and cell debris from urine by filtration or centrifugation, adding EDTA and Tris-HCl to urine, adding a chaotropic salt and a resin which binds the polynucleotide in the presence of the chaotropic salt, removing the resin from the urine and eluting the polynucleotide from the resin. Thereby, the polynucleotide is isolated from urine. Further information on this method can be taken from EP 2 351 857 A1, the content of which is incorporated herein by reference. Compositions, methods and kits for isolating polynucleotides from bodily fluids are also described in US 2008/139801 A1 and US 2010/068711 A1. Another example of polynucleotide isolation from urine is given in the Example section. Further information can be obtained from "The Nucleic Acid Protocols Handbook" by R. Rapley (2000, Springer), "RNA Methodologies: A Laboratory Guide for Isolation and Characterization" by R.E. Farrell, Jr. (2010, Academic Press) and "Molecular Diagnostics: Techniques and Applications for the Clinical Laboratory" by W.W. Grody et al. (2009, Academic Press).

The method step of amplifying the polynucleotides may be performed according to standard procedures and techniques well known to a person skilled in the art. Amplification of the polynucleotides can be performed *e.g.* by polymerase chain reaction or by reporter mediated amplification as disclosed in WO 2008/055915 A2. One example of polynucleotide amplification using real-time analysis and SYBR Green is given in the Example section. Further information in this respect can also be taken from before mentioned literature and patent references. Polynucleotide quantification can be performed e.g. using the DNA DipStick TM Kit from Life Technologies (formerly known as Invitrogen, Carlsbad, CA, USA) or using the PicoGreen assay (Molecular Probes, Netherlands). The method step of detecting the polynucleotides may be performed according to standard procedures and techniques well known to a person skilled in the art. For example via PCR real-time analysis and SYBR Green, the polynucleotide can be determined and detected. Principally, all fluorescent and/or radioactivity-based probes may be used for detecting the polynucleotide. Fluorescent probes may *e.g.* be obtained from Sigma-Aldrich (St. Louis, MO, USA). Detection and determination of polynucleotides can also be done by labeling the polynucleotides, e.g. labeling of miRNA using the mercury Hy3 power labeling kit (Exiqon, Vedbaek, Denmark), array hybridization and subsequent microarray data analysis. Using specific probes which can be designed using genomic sequence information of the respective pathogen, the polynucleotide can be specifically assigned to a pathogen. An exemplary probe is the repetitive insertion element IS6110 as specific target for the detection of the *Mycobacterium tuberculosis* Complex (MTC). Thereby, the pathogen infection can be diagnosed and the presence and the genotype of the pathogen can be determined specifically.

The assay or kit for performing the method disclosed herein comprises at least a device and/or means for isolating the polynucleotide from the bodily fluid, a device and/or means for amplifying the polynucleotide, and a device and/or means for detecting the polynucleotide. The device or the devices can be an automated system, e.g. integrated into an automated system, performing the method steps as described above or in the references given. The device for amplifying and detecting the polynucleotide may be a real-time PCR machine or microarray. The devices and methods described in WO 2005/108604 regarding detecting specific interactions between probe and target molecules and microarray techniques are explicitly incorporated herein by reference. In another aspect, the method is compatible with the assays, methods and devices described in WO 2009/013321 A2.

### Examples

### 1. Study Population and Materials and Methods

### 1.1 Study participants

Ethical approval was obtained from the Ethics Committee of the University of Luebeck, Germany. Informed consent was obtained from all participants.

### 1.1.1 Patient enrolment

Study participants were adults (>18 years) enrolled at the FZB Borstel, Medical Hospital (Clinic), Borstel, Germany. The patients were under hospital care to clarify the tuberculosis (TB) suspicion. TB patients were finally diagnosed as lung TB positive on the basis of microbiological tests (direct microscopy and culture) out of sputum and clinical findings (chest X-ray, clinical symptoms). All patients were treatment naive (t=0) at the beginning of the study. The start of anti-TB therapy and the medication (antibiotics and dosages) were decided by the physician in charge of the patient. Patients were treated as follows: pyrazinamide (Pyrofat, Junek Europ V. GmbH Zn or pyrazinamide generics, Riemser Arzneimittel AG, dosis 35 mg/kg body weight, 2 g, p.o.), ethambutol (EMB-Fatol, Riemser Arzneimittel AG, or EMB-Hefa, Sanav.Werniger, myambutol, Emra-Med Arzneimittel GmbH, dosis 25 mg/kg body weight p.o., rifampicin (Eremfat, Riemser Arzneimittel AG, or Rifa, Riemser Arzneimittel AG, dosis 10 mg/kg body weight, 600 mg p.o. and isoniazid (isoniazid generics, Pfizer Limited, or Macleods Pharmaceuticals Pvt Ltd,or Cyper Pharma, dosis 5 mg/kg body weight, 300 mg p.o. Exclusion criteria: urogenital tuberculosis, systemic tuberculosis (sepsis).

### 1.2 Sample collection

The sample collection (urine, EDTA blood, sputum) was started at time (t=0) of TB suspicion before beginning the anti-TB treatment and continued until the patient discharge from hospital. All patients donated a cup (ca. 50 mL) of morning urine two times a week until week 4 and then weekly while hospitalized. Ten mL EDTA blood was drawn weekly during routine care. Sputum samples were collected weekly for the diagnostic laboratory to perform the routine microbiological testing (microscopy and culture).

### 1.3 Laboratory methods

### 1.3.1 Urine processing and parameter determination

EDTA was added immediately after urine collection to avoid nucleic acid degradation at a final concentration of 25 mM. Combur10 test (F. Hoffmann-La Roche, Basel, Switzerland) was used to perform semi-quantitative determination of following parameters: specific gravity (SG), pH, leukocytes (LEU), nitrite (NIT), protein (PRO), glucose (GLU), ketone bodies (KET), urobilinogen (UBG), bilirubin (BIL), and blood (ERY and Hb) in urine.

### 1.3.2 Nucleic acid isolation from urine

Total nucleic acids were isolated out of 4 mL of unfractionated urine. Briefly, 1 mL of silica matrix was added to 6 mL of binding solution containing the chaotropic salt guanidine thioisocyanate (6 M). Then 4 mL of urine were added to the binding matrix solution and gently mixed by head-over-head rotation (pipetting). For total DNA binding to the silica matrix no further incubation step was necessary. Following the DNA capture step the sample-matrix solution was applied on to a mini spin column using a 10 mL syringe over a luer-lock connection. The silica-DNA matrix was separated from the residual solution by pushing the plunger and the flow-through collected in a waste tube.

The silica matrix was washed twice with 400 µL washing buffer (80 mM potassium acetate, 8.3 mM Tris-HCl, 40 µM EDTA, 55% EtOH; pH 7.5) and spinning at 8000 rpm for 1 min to remove inhibitory sample or binding buffer components. After the washing steps the column was transferred to a fresh tube and spun for 2 min at 10.000 rpm to dry the silica matrix and remove residual ethanol. Then the spin column was transferred in a DNA low-binding tube, 30 µL nuclease-free water was added and the sample incubated for 1 min at room temperature. The first fraction of DNA was eluted by spinning at 16.000 g for 1 min. To increase the efficiency of the DNA elution, further 20 µL nuclease-free water were added to the silica matrix, incubated for another minute and then spun at high speed. The DNA fraction was then stored at -80 °C until PCR analysis. All experiments were run in duplicate.

### 1.3.3 Plasma processing and nucleic acid isolation from plasma

10 mL EDTA blood was spun for 10 min at 800 g to separate the plasma from blood cells. The plasma was transferred in a 2 mL cryo tube and stored at -80 °C until processing.

Circulating nucleic acids were isolated out of 1 mL plasma using the Qiagen Amp Circulating Nucleic Acid Isolation Kit (Qiagen, Hilden, Germany). The captured DNA was eluted with 50 µL of nuclease-free water and the DNA stored at -80 °C until PCR analysis. All experiments were run in duplicate.

### 1.3.4 Real-Time PCR analysis using SYBR Green

The repetitive insertion element IS6110 was chosen as specific target for the detection of the *Mycobacterium tuberculosis* Complex (MTC). Plasma and urine from patients with pulmonary TB can contain degradation products, *i.e.* DNA that results from metabolic active or degraded bacteria in the circulation. DNA fragments from different length in plasma and in urine. In urine, the polynucleotides are about 100 bp or even smaller. Thus, the IS6110 amplicon size was chosen at 38 bp for the detection of MTC-specific nucleic acids in plasma and urine. For the detection of the 38 bp amplicon, a SYBR Green based Real-Time PCR assay was performed in a final reaction volume of 100 µL with 20 µL isolated DNA. The PCR reaction mix contained 300 nmol of each primer (forward primer: pfw_sybr:IS6110:315, reverse primer: prv_63mer:IS6110:311), 1-fold SYBR Green Mastermix (Qiagen, Hilden, Germany) and 20 µL of isolated DNA. The heat activation step was carried out for 15 min at 95 °C followed by 40 cycles of 15 s at 95 °C for denaturation, 30 s at 62 °C for annealing and 30 s at 72 °C for elongation. Melting curve analysis followed a two-stage with 15 s 95 °C, 60 s 60 °C ramp to 95 °C (3% gradient). Other possible primer pairs for this real-time PCR analysis are: forward: pfw_sybr:IS6110:320 and reverse primer: prv_sybr:IS6110:321; forward: pfw_sybr_IS1081_314 and reverse primer: prv_62mer_IS1081_311. Further reverse primer which may be used in the before-mentioned pairs are prv_63mer:IS6110:311 and prv_62mer_IS1081_311.

Specific gravity was used for normalization of determined nucleic acid measurements in urine.

### 2. Results

### Patient 22

Sample Collection (sputum, urine) was started at day 1 after treatment initiation. Sputum samples were collected weekly until day 29. Results from the routine direct microscopy and liquid culture confirmed lung TB diagnosis. Direct microscopy was done using Ziehl-Nelsen (ZN) staining. The microscopy results are classified according to an international score: 0 (negative), 1(4-49 bacteria/100 fov(field of view)), 2 (5-49 bacteria/10 fov), 3 (5-49 bacteria/1 fov) and 4 (>49 bacteria/1 fov) performed until day 29. The results from mycobacterial culture are given as days to culture positivity, performed until day 15. Molecular typing classified the *Mycobacterium tuberculosis* Complex (MTC) strain as *Mycobacterium africanum,* no resistance was detected.

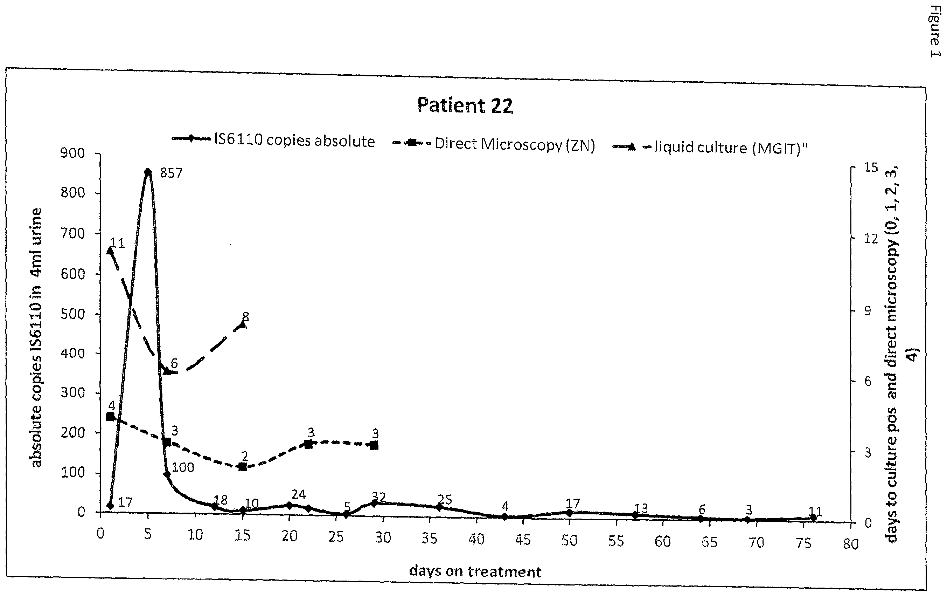

Morning urine samples were collected twice weekly over the first 4 weeks (day 1, 5, 7, 12, 15, 20, 22, 26, 29) and then weekly until day 76. Total DNA was isolated as outlined in chapter 1.3 from 4 mL unfractionated urine and the 38 bp IS6110 target amplified using SYBR Green PCR. One day after treatment initiation, the detected trDNA amount was very low with 17 single copies of IS6110 38 bp amplicon (at LOD level). At day 5 of treatment, *i.e.* day 5 prior to obtaining and/or isolating the polynucleotides from the bodily fluid, an enormous increase in trDNA was measured reaching its maximum with 857 single copies. The analysis of the isolated DNA from the consecutive days until day 12 shows a rapid decline in the trDNA amount to a basic level of 18 copies. Detectable trDNA then steadily decreases and gains negative at day 69 upon antibiotic treatment. The detected peak of measurable MTC DNA at day 5 reflects the effectiveness of mycobacterial killing induced by the administered antibiotics. All data are normalized to the specific gravity in urine. Molecular typing classified MTC strain as *Mycobacterium africanum,* no resistance was detected. These results are presented in **Figure 1****.**

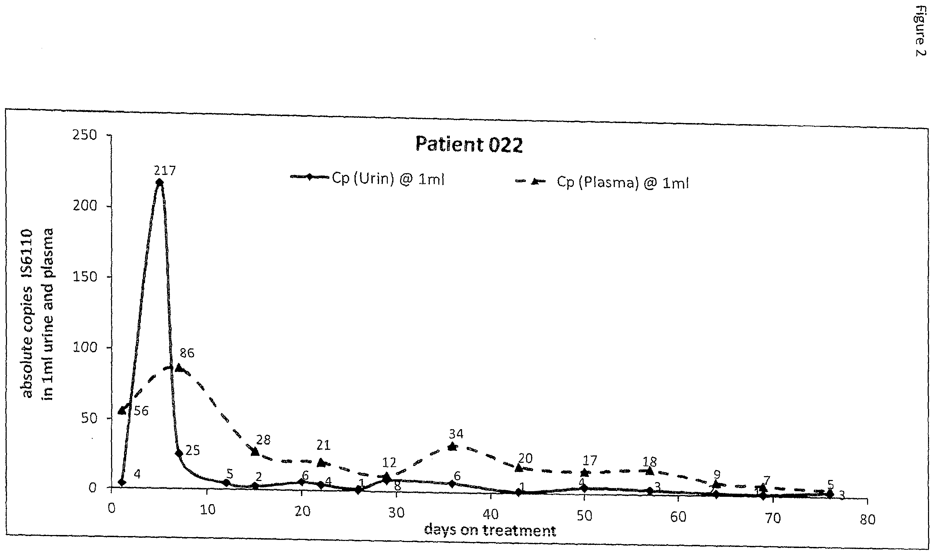

Morning urine and 10 mL EDTA blood were started to collect at day 1 of treatment initiation until day 76. EDTA blood was drawn weekly within a routine care. Plasma was separated from blood cells immediately after blood collection and banked until PCR analysis at -80 °C. DNA was isolated out of 1 mL plasma using the QIAamp Circulating Nucleic Acid Isolation Kit (Qiagen, Hilden, Germany) according to the manufacturer's instructions. Urine was collected twice weekly until week 4 and then weekly until week 12 (day 76). DNA was isolated out of 4 mL urine as outlined in chapter 1.3. For amplification of the 38 bp target region specific for the IS6110 insertion element as mentioned above, 20 µL of the isolated DNA from urine or plasma were amplified using SYBR Green PCR. Figure 2 presents the detected amount of circulating nucleic acids in 1 mL plasma and of trDNA in 1 mL urine. The maximum amount of trDNA in urine is reached at day 5 upon antibiotic treatment with 217 copies of IS6110 38 bp amplicon. Then a steadily decrease in trDNA is measured getting negative at day 43. In plasma, the MTC specific IS6110 38 bp amplicon is detectable, but in a lower concentration values compared to urine, probably due to up-concentration in urine. At day 1 after treatment initiation, 56 copies IS6110 38 bp are detected in plasma. The maximum peak of circulating nucleic acids is measured at day 7 with 86 copies. Within the following days the DNA amount declined to a basic level of 21 copies getting negative at day 76. These results are presented in **Figure 2****.**

It can be seen from the data presented in **Figure 1** and **Figure 2** that pathogen polynucleotides, in particular polynucleotides derived from *Mycobacterium tuberculosis,* can be detected in an early phase as response on antibiotic treatment initiation ("early response peak"). In this particular lung TB patient, mycobacterial polynucleotides were measured in plasma (circulating polynucleotides) and urine (transrenal polynucleotides) in highest concentration between day 1 and day 10 (here peak at day 5) upon administration of anti-pathogenic agent, *i.e*. antibiotic. The TB-specific anti-pathogenic agent induced the killing of *Mycobacterium tuberculosis* and the release of mycobacterial polynucleotides in the blood circulation within the first week of therapy (circulating polynucleotides).

This effect resulted in an increased level of circulating polynucleotides detectable as early response peak which could be used to diagnose TB infection. A treatment-induced diagnosis of TB could also be done very efficiently in urine reaching higher levels (absolute concentration) compared to plasma. Approximately 5% of the circulating DNA in the circulation generally passes the renal barrier of kidney to end up in urine in slightly concentrated form. In urine and in blood/plasma/serum, it is possible to detect a pathogen in response to an anti-pathogenic agent in an early phase of an infectious disease and evaluate the efficiency of the pathogenic agent with respect to the specific pathogen. In response thereto, the anti-pathogenic agent may be altered, e.g. selected more specifically, and/or dosage may be changed.

### Patients 50 and 56:

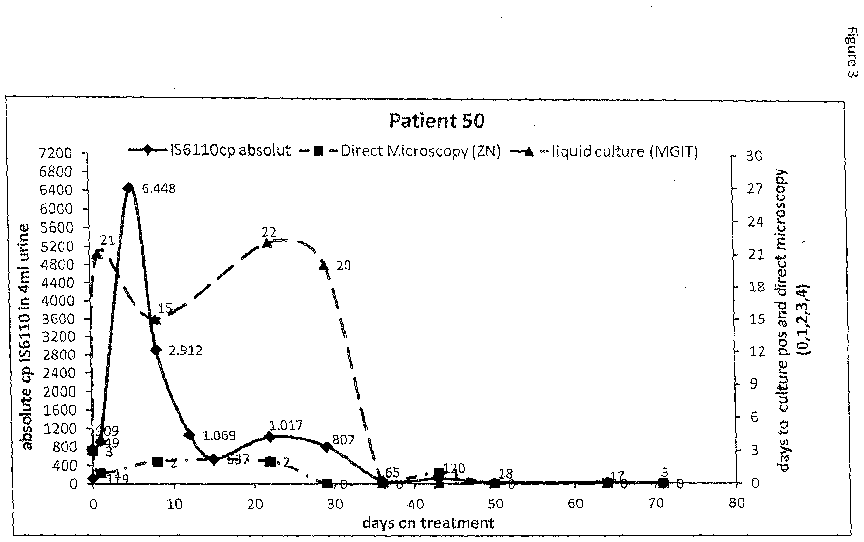

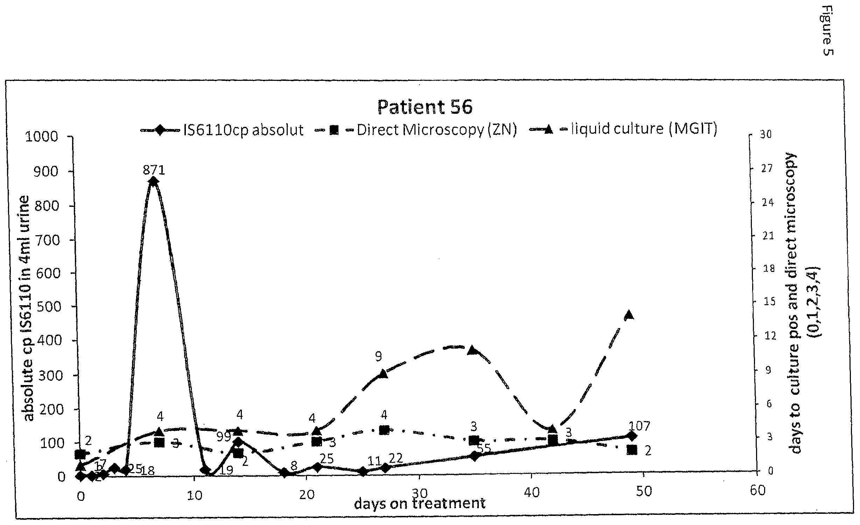

Sample collection (sputum, urine, blood) for patients 50 and 56 were started prior to treatment initiation at day 0. Sputum samples were collected weekly until day 71 (patient 50) and 49 (patient 56). Results from the routine direct microscopy and liquid culture confirmed lung TB diagnosis. Direct microscopy was done using Ziehl-Nelsen (ZN) staining. The microscopy results are classified according to an international score: 0 (negative), 1(4-49 bacteria/100 fov(field of view)), 2 (5-49 bacteria/10 fov), 3 (5-49 bacteria/1 fov) and 4 (>49 bacteria/1 fov) performed until day 29. The results from mycobacterial culture are given as days to culture positivity, performed until day 64 (patient 50) and 49 (patient 56). Molecular typing classified the *Mycobacterium tuberculosis* Complex (MTC) strains as *Mycobacterium tuberculosis,* no resistance was detected (see **Figure 3** and **Figure 5**).

Morning urine samples were collected at multiple time points until day 22 (0, 1, 5, 8, 12, 15, 22 for patient 50) and day 25 (0, 1, 2, 3, 4, 7, 11, 14, 18, 21, 25) and then weekly until day 71 (patient 50) and day 49 (patient 56). Total DNA was isolated as outlined in chapter 1.3 from 4 mL unfractionated urine and the 38 bp IS6110 target amplified using SYBR Green PCR. Prior treatment initiation, the detected trDNA amount was low with 119 single copies of IS6110 38 bp amplicon (patient 50) and non trDNA was detected for patient 56. At day 5 of treatment, *i.e.* day 5 prior to obtaining and/or isolating the polynucleotides from the bodily fluid, an enormous increase in trDNA was measured for patient 50 reaching its maximum with 6448 single copies. The analysis of the isolated DNA from the consecutive days until day 43 shows a rapid decline in the trDNA amount to a basic level of 17 copies. Detectable trDNA then steadily decreases and gains negative at day 71 upon antibiotic treatment. This reflects the effective elimination of the mycobacteria from the circulation which is confirmed by negative results in the sputum (direct microscopy and liquid culture). Patient 56 shows a similar kinetics in trDNA excretion reaching its maximum at day 7 with 871 single copies. During the consecutive days the level of trDNA is steadily falling. Contrary to patient 50 the trDNA measurement is still positive at day 49 as well as the sputum samples (direct microscopy and liquid culture). The detected peaks of measurable MTC DNA at day 5 and 7 reflect the effectiveness of mycobacterial killing induced by the administered antibiotics. All data are normalized to the specific gravity in urine. Molecular typing classified MTC strain as *Mycobacterium tuberculosis,* no resistance was detected. These results are presented in **Figure 3** **and** **5****.**

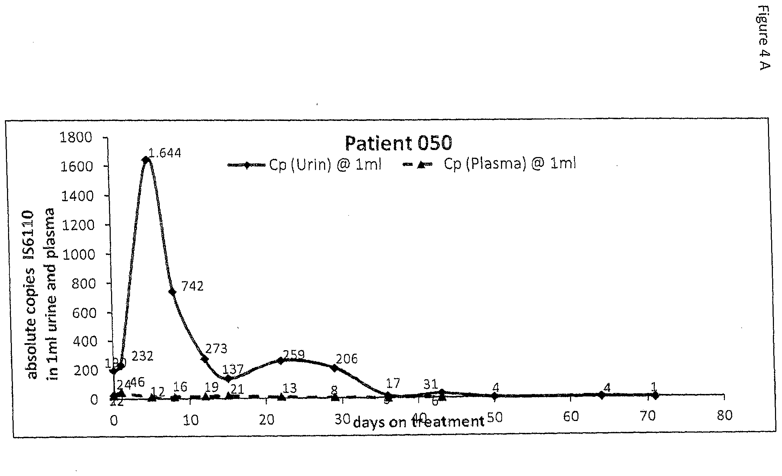

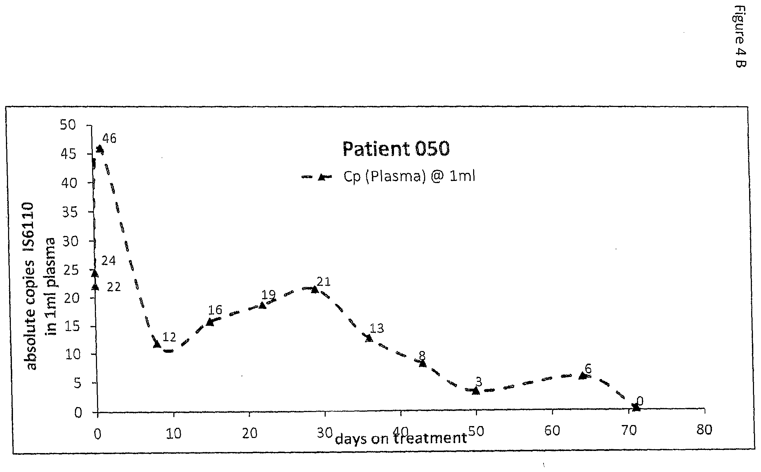

Morning urine and 10 mL EDTA blood were started to collect prior to treatment initiation at day 0 until day 71 (patient 50) and 49 (patient 56). EDTA blood was drawn weekly within a routine care. Plasma was separated from blood cells immediately after blood collection and banked until PCR analysis at -80 °C. DNA was isolated out of 1 mL plasma using the QIAamp Circulating Nucleic Acid Isolation Kit (Qiagen, Hilden, Germany) according to the manufacturer's instructions. Urine was collected as described before (fig 3 and 5). DNA was isolated out of 4 mL urine as outlined in chapter 1.3. For amplification of the 38 bp target region specific for the IS6110 insertion element as mentioned above, 20 µL of the isolated DNA from urine or plasma were amplified using SYBR Green PCR. Figure 4 (patient 50) presents the detected amount of trDNA in 1 mL urine (4A) and of circulating nucleic acids in 1 mL plasma (4B). The maximum amount of trDNA in urine is reached at day 5 upon antibiotic treatment with 1644 copies of IS6110 38 bp amplicon. Then a steadily decrease in trDNA is measured getting negative at day 71. In plasma, the MTC specific IS6110 38 bp amplicon is detectable, but in a lower concentration values compared to urine, probably due to up-concentration in urine.

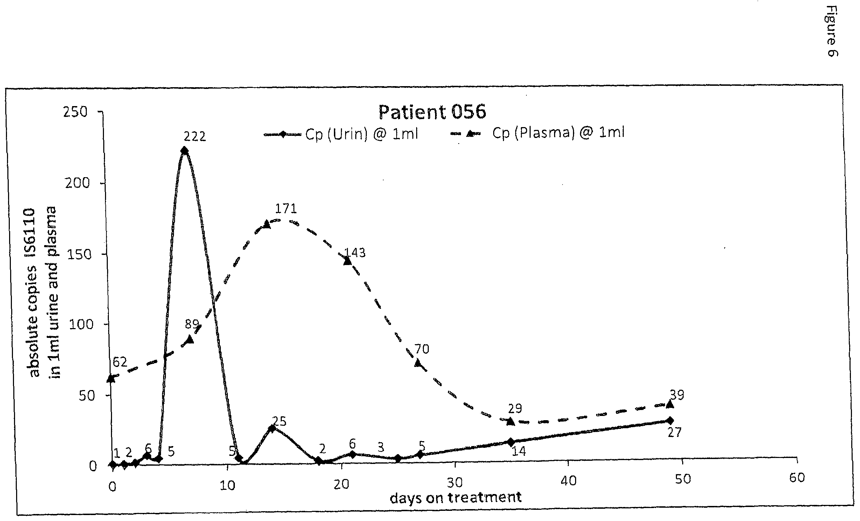

Prior to treatment initiation, 22 copies IS6110 38 bp are detected in plasma. The maximum peak of circulating nucleic acids is measured at day 1 with 46 copies. Within the following days the DNA amount declined getting negative at day 71 (Figure 4). Figure 6 presents the detected amount of trDNA in 1ml urine and of circulating nucleic acids in 1ml plasma for patient 56. The maximum of trDNA in urine is reached at day 7 with 222 single copies, then it steadily decreases, however still remains positive at day 49 as bacteria are still found in the sputum (figure 5). In plasma, the kinetics differs from patient 5. At day 0 the base level is low with 62 copies. Then the DNA amount increases reaching its peak at day 14 with 171 single copies, 7 days later then in urine the max value was detected. During the consecutive measurements the DNA is steadily declining reaching a basic level between 30 and 40 copies to at day 49 which corresponds to positive MTC detection in urine and sputum.

It can be seen from the data presented in **Figure 4** and **Figure 6** that pathogen polynucleotides, in particular polynucleotides derived from *Mycobacterium tuberculosis,* can be detected in an early phase as response on antibiotic treatment initiation ("early response peak"). In these particular lung TB patients, mycobacterial polynucleotides were measured in plasma (circulating polynucleotides) and urine (transrenal polynucleotides) in highest concentration between day 1 and day 14 upon administration of anti-pathogenic agent, *i.e.* antibiotic. The TB-specific anti-pathogenic agent induced the killing of *Mycobacterium tuberculosis* and the release of mycobacterial polynucleotides in the blood circulation within the first 2 weeks of therapy (circulating polynucleotides).

This effect resulted in an increased level of circulating polynucleotides detectable as early response peak which could be used to diagnose TB infection. A treatment-induced diagnosis of TB could also be done efficiently in urine reaching higher levels (absolute concentration) compared to plasma. Approximately 5% of the circulating DNA in the circulation generally passes the renal barrier of kidney to end up in urine in slightly concentrated form. In urine and in blood/plasma/serum, it is possible to detect a pathogen in response to an anti-pathogenic agent in an early phase of an infectious disease and evaluate the efficiency of the pathogenic agent with respect to the specific pathogen. In response thereto, the anti-pathogenic agent may be altered, e.g. selected more specifically and/or dosage may be changed.

### Patient 21:

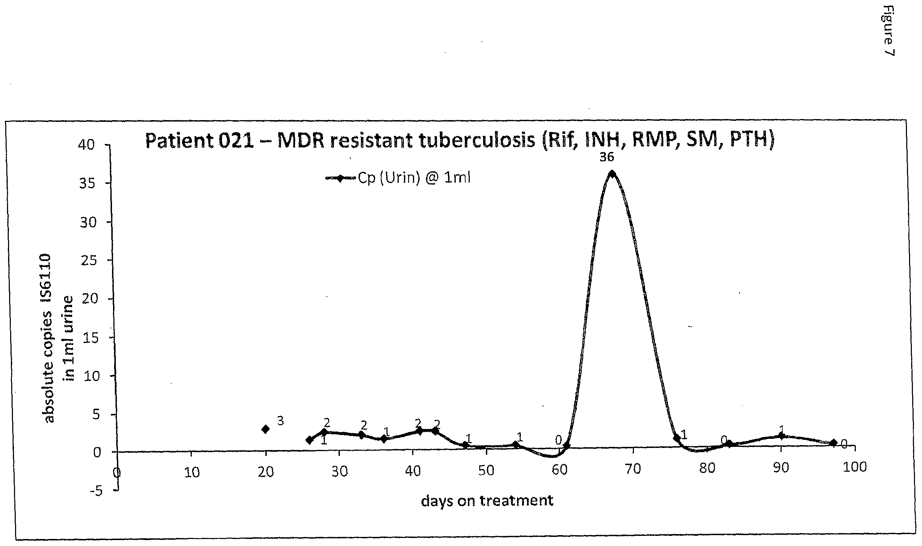

Morning urine samples from patient 21 were collected twice weekly from day 20 (day 20, 22, 26, 28, 33, 36, 41, 43) to 43 and then weekly until day 97. Total DNA was isolated as outlined in chapter 1.3 from 4 mL unfractionated urine and the 38 bp IS6110 target amplified using SYBR Green PCR. Patient received standard first-line therapy (Rif, INH, PZA, ETB). Molecular typing classified MTC strain as *Mycobacterium tuberculosis.* Drug susceptibility testing detected resistance to Rifampicin (Rif), Isoniazid (INH), RMP, Streptomycin (SM) and Prothionamid (PTH). Thus, therapy regiment was changed at day 63 to PAS (para-aminosalicylic acid), administered intravenously (iv). Five days after PAS treatment, trDNA excretion was detected in urine with 144 single copies (36 copies per ml urine) maximum at day 68. The analysis of DNA from the following days until day 97 was negative like the measurements upon standard first-line treatment. The detected peak of measurable MTC DNA at day 68 reflects the effectiveness of mycobacterial killing induced by the administered PAS. All data are normalized to the specific gravity in urine. These results are presented in **Figure 7****.**

### Patient 23:

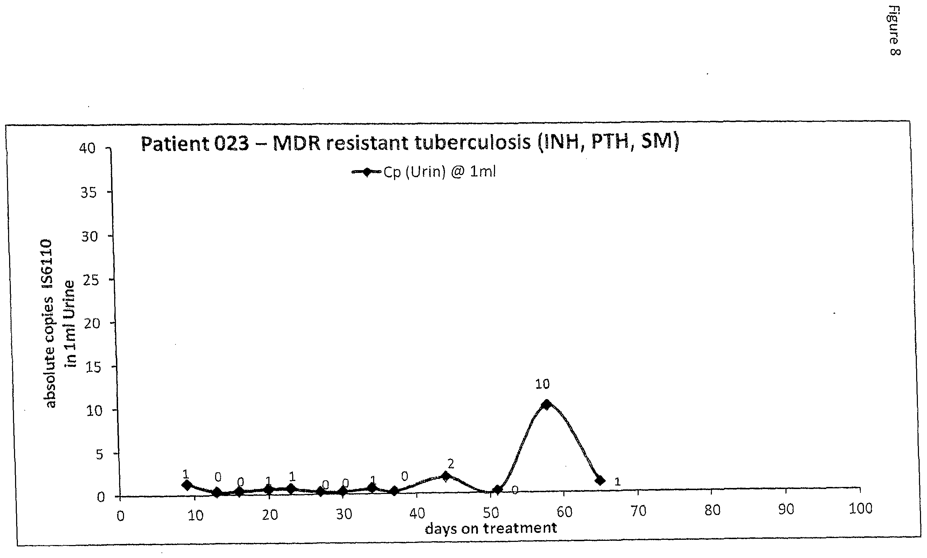

Morning urine samples were collected twice weekly from day 9 (day 9, 13, 16, 20, 23, 27, 30, 34, 37, 44, 51, 58, 65) to 34 and then weekly until day 65. Total DNA was isolated as outlined in chapter 1.3 from 4 mL unfractionated urine and the 38 bp IS6110 target amplified using SYBR Green PCR. Patient received standard first-line therapy (Rif, INH, PZA, ETB). Molecular typing classified MTC strain as *Mycobacterium tuberculosis.* Drug susceptibility testing detected resistance to Isoniazid (INH), Streptomycin (SM) and Prothionamid (PTH). Thus, therapy regiment was changed at day 38 to a new regiment composed of Ethambutol (ETB), Pyrazinamid (PZN), Moxi, Capreo and Terizidon. Twenty days after treatment modification, trDNA excretion was detected in urine with 40 single copies (10 copies per ml urine) max at day 58. The analysis of DNA from the following urine sample at day 65 was negative as the measurements upon standard first-line treatment. The detected peak of measurable MTC DNA at day 58 reflects the effectiveness of mycobacterial killing induced by the treatment modification. All data are normalized to the specific gravity in urine. These results are presented in **Figure 8****.**

It can be seen from the data presented in **Figure 7** and **Figure 8** that pathogen polynucleotides, in particular polynucleotides derived from *Mycobacterium tuberculosis,* can be detected in response to treatment modification in cases of multidrug resistant tuberculosis (response peak to effective drug regiment).

## Claims

1. A method of diagnosing or monitoring a pathogen infection selected from the group consisting of tuberculosis, pulmonary tuberculosis, paediatric, extrapulmonary or HIV-associated tuberculosis in a subject, comprising:
determining a maximum of a value indicative of the presence and/or amount of a polynucleotide indicative of the pathogen infection in a sample of a bodily fluid obtained from the subject 1 to 10 days after administration of an anti-pathogenic agent to the subject, wherein the bodily fluid is selected from the group consisting of urine, blood, plasma and serum, wherein the anti-pathogenic agent is effective against a pathogen of the genus Mycobacterium, and wherein the polynucleotide is released from a dead pathogenic cell; and
comparing the value to a baseline value indicative of the presence and/or amount of the polynucleotide in a sample of the bodily fluid before administration of the anti-pathogenic agent, wherein a value higher than the baseline value is indicative of a pathogen infection.

2. The method of claim 1,
wherein the baseline value is determined based on a sample of the bodily fluid obtained from the same subject or from a population of subjects.

3. The method of claim 1 or 2, comprising
- isolating a polynucleotide indicative of the pathogen infection from a first sample of a bodily fluid obtained from the subject before administration of an anti-pathogenic agent to the subject,
- determining a baseline value indicative of the presence and/or amount of the polynucleotide in the first sample of the bodily fluid,
- isolating the polynucleotide indicative for the pathogen infection from a second sample of the bodily fluid obtained from the subject after administration of the anti-pathogenic agent,
- determining a value indicative of the presence and/or amount of the polynucleotide in the second sample of the bodily fluid, and
- comparing the value to the baseline value, wherein a value higher than the baseline value is indicative of a pathogen infection.

4. The method of any one of claims 1 to 3, wherein the polynucleotide is a microbial polynucleotide.

5. The method of claim 4, wherein the pathogen polynucleotide is a bacterial polynucleotide.

6. The method of any one of the preceding claims, wherein the polynucleotide is DNA or RNA.

7. The method of any one of the preceding claims, wherein the DNA or RNA is renal or transrenal DNA, renal or transrenal RNA, or microRNA.

8. The method of any one of the preceding claims, wherein the polynucleotide is derivable from a pathogen selected from the group consisting of Mycobacterium tuberculosis, Mycobacterium bovis, Mycobacterium africanum, and combinations thereof.

9. The method of any one of the preceding claims, wherein the subject is a human or animal.

10. The method of any one of the preceding claims, wherein the subject was treated with the anti-pathogenic agent for 2 to 8 days, prior to obtaining the sample of the bodily fluid and/or isolating the polynucleotide from the sample of the bodily fluid.

11. The method of any one of the preceding claims, wherein the anti-pathogenic agent is an antibiotic, optionally a natural antibiotic or an antibiotic analog, wherein the antibiotic analog is optionally a synthetic antibiotic or a semi-synthetic antibiotic.

12. The method of any one of the preceding claims, wherein the anti-pathogenic agent is selected from the group consisting of isoniazid, rifampicin, pyrazinamide, ethambutol, streptomycin, and combinations thereof.

13. The method of any of the preceding claims, further comprising determining the genotype of the pathogen.

## Patentansprüche

1. Verfahren zum Diagnostizieren oder Monitoren einer pathogenen Infektion ausgewählt aus der Gruppe bestehend aus Tuberkulose, Lungentuberkulose, pädiatrische Tuberkulose, Tuberkulose außerhalb der Lunge oder HIV-assoziierter Tuberkulose in einem Subjekt, umfassend:
Bestimmen eines Maximums eines Werts indikativ für die Anwesenheit und/oder Menge eines Polynukleotids indikativ für die pathogene Infektion in einer Körperflüssigkeitprobe, die von dem Subjekt 1 bis 10 Tagen nach Verabreichung eines antipathogenen Mittels an das Subjekt erhalten wurde, wobei die Körperflüssigkeit ausgewählt ist aus der Gruppe bestehend aus Urin, Blut, Plasma und Serum, wobei das antipathogene Mittel wirksam gegen ein Pathogen des Genus Mycobacterium ist und wobei das Polynukleotid von einer toten pathogenen Zelle freigesetzt wird; und
Vergleichen des Werts zu einem Baseline-Wert indikativ für die Anwesenheit und/oder Menge des Polynukleotids in einer Körperflüssigkeitprobe vor Verabreichung des antipathogenen Mittels, wobei ein Wert höher als der Baseline-Wert indikativ für eine pathogene Infektion ist.

2. Verfahren gemäß Anspruch 1,
wobei der Baseline-Wert bestimmt wird basierend auf einer Körperflüssigkeitprobe, die von dem gleichen Subjekt erhalten wurde oder von einer Gruppe von Subjekten.

3. Verfahren gemäß Anspruch 1 oder 2, umfassend
- Isolieren eines Polynukleotids indikativ für die pathogene Infektion von einer ersten Körperflüssigkeitprobe, die von dem Subjekt vor Verabreichung eines antipathogenen Mittels an das Subjekt erhalten wurde,
- Bestimmen eines Baseline-Wertes indikativ für die Anwesenheit und/oder Menge des Polynukleotids in der ersten Körperflüssigkeitprobe,
- Isolieren des Polynukleotids indikativ für die pathogene Infektion von einer zweiten Körperflüssigkeitprobe, die von dem Subjekt nach Verabreichung des antipathogenen Mittels erhalten wurde,
- Bestimmen eines Werts indikativ für die Anwesenheit und/oder Menge des Polynukleotids in der zweiten Körperflüssigkeitprobe, und
- Vergleichen des Werts mit dem Baseline-Wert, wobei ein Wert höher als der Baseline-Wert indikativ für eine pathogene Infektion ist.

4. Verfahren gemäß einem der Ansprüche 1 bis 3, wobei das Polynukleotid ein mikrobielles Polynukleotid ist.

5. Verfahren gemäß Anspruch 4, wobei das pathogene Polynukleotid ein bacterielles Polynukleotid ist.

6. Verfahren gemäß einem der vorstehenden Ansprüche, wobei das Polynukleotid DNA oder RNA ist.

7. Verfahren gemäß einem der vorstehenden Ansprüche, wobei die DNA oder RNA renale oder transrenale DNA, renale oder transrenale RNA, oder MikroRNA ist.

8. Verfahren gemäß einem der vorstehenden Ansprüche, wobei das Polynukleotid von einem Pathogen ausgewählt aus der Gruppe bestehend aus Mycobacterium tuberkulosis, Mycobacterium bovis, Mycobacterium africanum und Kombinationen davon abgeleitet ist.

9. Verfahren gemäß einem der vorstehenden Ansprüche, wobei das Subjekt ein Mensch oder Tier ist.

10. Verfahren gemäß einem der vorstehenden Ansprüche, wobei das Subjekt mit dem antipathogenen Mittel für 2 bis 8 Tage behandelt wurde, bevor die Körperflüssigkeitprobe erhalten wurde und/oder das Polynukleotid von der Körperflüssigkeitprobe isoliert wurde.

11. Verfahren gemäß einem der vorstehenden Ansprüche, wobei das antipathogene Mittel ein Antibiotikum, optional ein natürliches Antibiotikum oder ein Antibiotikum-Analog ist, wobei das Antibiotikum-Analog optional ein synthetisches Antibiotikum oder ein halbsynthetisches Antibiotikum ist.

12. Verfahren gemäß einem der vorstehenden Ansprüche, wobei das antipathogene Mittel ausgewählt aus der Gruppe bestehend aus Isoniazid, Rifampicin, Pyrazinamid, Ethambutol, Streptomycin und Kombinationen davon ist.

13. Verfahren gemäß einem der vorstehenden Ansprüche, ferner umfassend Bestimmen des Genotyps des Pathogens.

## Revendications

1. Procédé de diagnostic ou de suivi d'une infection pathogène sélectionnée dans le groupe constitué par la tuberculose, la tuberculose pulmonaire, la tuberculose pédiatrique, extrapulmonaire ou associée au VIH chez un sujet, comprenant :
la détermination d'un maximum d'une valeur indicatrice de la présence et/ou de la quantité d'un polynucléotide indicateur de l'infection pathogène dans un échantillon d'un fluide corporel obtenu à partir du sujet 1 à 10 jours après administration au sujet d'un agent antipathogène, sachant que le fluide corporel est sélectionné dans le groupe constitué par l'urine, le sang, le plasma et le sérum, sachant que l'agent antipathogène est effectif contre un pathogène du genre Mycobacterium, et sachant que le polynucléotide est libéré à partir d'une cellule pathogène morte ; et
la comparaison de la valeur à une valeur de base indicatrice de la présence et/ou de la quantité du polynucléotide dans un échantillon du fluide corporel avant administration de l'agent pathogène, sachant qu'une valeur plus élevée que la valeur de base est indicatrice d'une infection pathogène.

2. Le procédé de la revendication 1,
sachant que la valeur de base est déterminée sur la base d'un échantillon du fluide corporel obtenu à partir du même sujet ou à partir d'une population de sujets.

3. Le procédé de la revendication 1 ou 2, comprenant
- l'isolation d'un polynucléotide indicateur de l'infection pathogène à partir d'un premier échantillon d'un fluide corporel obtenu à partir du sujet avant administration d'un agent antipathogène au sujet,
- détermination d'une valeur de base indicatrice de la présence et/ou de la quantité du polynucléotide dans le premier échantillon du fluide corporel,
- isolation du polynucléotide indicateur de l'infection pathogène à partir d'un deuxième échantillon du fluide corporel obtenu à partir du sujet après administration de l'agent antipathogène,
- détermination d'une valeur indicatrice de la présence et/ou de la quantité du polynucléotide dans le deuxième échantillon du fluide corporel, et
- comparaison de la valeur à la valeur de base, sachant qu'une valeur plus élevée que la valeur de base est indicatrice d'une infection pathogène.

4. Le procédé de l'une quelconque des revendications 1 à 3, sachant que le polynucléotide est un polynucléotide microbien.

5. Le procédé de la revendication 4, sachant que le polynucléotide pathogène est un polynucléotide bactérien.

6. Le procédé de l'une quelconque des revendications précédentes, sachant que le polynucléotide est de l'ADN ou de l'ARN.

7. Le procédé de l'une quelconque des revendications précédentes, sachant que l'ADN ou l'ARN est de l'ADN rénal ou transrénal, de l'ARN rénal ou transrénal, ou du microARN.

8. Le procédé de l'une quelconque des revendications précédentes, sachant que le polynucléotide est dérivable d'un pathogène sélectionné dans le groupe constitué par Mycobacterium tuberculosis, Mycobacterium bovis, Mycobacterium africanum, et leurs combinaisons.

9. Le procédé de l'une quelconque des revendications précédentes, sachant que le sujet est un humain ou un animal.

10. Le procédé de l'une quelconque des revendications précédentes, sachant que le sujet a été traité avec l'agent antipathogène pendant 2 à 8 jours, avant d'obtenir l'échantillon du fluide corporel et/ou d'isoler le polynucléotide de l'échantillon du fluide corporel.

11. Le procédé de l'une quelconque des revendications précédentes, sachant que l'agent antipathogène est un antibiotique, facultativement un antibiotique naturel ou un analogue d'antibiotique, sachant que l'analogue d'antibiotique est facultativement un antibiotique synthétique ou un antibiotique semisynthétique.

12. Le procédé de l'une quelconque des revendications précédentes, sachant que l'agent antipathogène est sélectionné dans le groupe constitué par l'isoniazide, la rifampicine, la pyrazinamide, l'éthambutol, la streptomycine, et leurs combinaisons.

13. Le procédé de l'une quelconque des revendications précédentes, comprenant en outre la détermination du génotype du pathogène.
